# EUROPÄISCHE PATENTSCHRIFT

(11) **EP 0 801 651 B1**
(45) Veröffentlichungstag und Bekanntmachung des Hinweises auf die Patenterteilung: **11.07.2001**
(21) Anmeldenummer: 96900024.9
(22) Anmeldetag: 03.01.1996
(51) Int. Cl.: C07D 495/14, A61K 31/55, C07D 487/04

(54) **HYDROXYMETHYL-IMIDAZODIAZEPINE UND DEREN ESTER**
HYDROXYMETHYLIMIDAZODIAZEPINES AND THEIR ESTERS
HYDROXYMETHYLIMIDAZODIAZEPINES ET LEURS ESTERS

(30) Priorität: 06.01.1995 CH 2895
(43) Veröffentlichungstag der Anmeldung: 22.10.1997
(73) Patentinhaber: F. HOFFMANN-LA ROCHE AG, 4002 Basel (CH)
(72) Erfinder: BORER, René, CH-4153 Reinach (CH); BÜTTELMANN, Bernd, D-79650 Schopfheim (DE); GERECKE, Max, CH-4153 Reinach (CH); SZENTE, André, CH-4125 Riehen (CH)
(74) Vertreter: Vergani, Diego, Dr.
(86) Internationale Anmeldenummer: CH9600001
(87) Internationale Veröffentlichungsnummer: WO9620941

(56) Entgegenhaltungen:
- EP-A- 0 139 460
- US-A- 4 280 957
- US-A- 4 902 794

## Beschreibung

Die vorliegende Erfindung betrifft Hydroxymethyl-imidazodiazepine und deren Ester der allgemeinen Formel 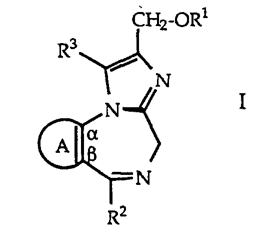 worin A zusammen mit den beiden mit α und β bezeichneten Kohlenstoffatomen einen der Reste 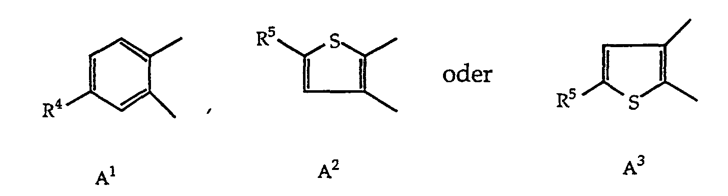
- R¹: Wasserstoff oder nieder-Alkanoyl;
- R²: Phenyl, o-Halophenyl oder 2-Pyridyl;
- R³: Wasserstoff, nieder-Alkyl, Methylaminomethyl, Allylaminomethyl oder Diethylaminomethyl;
- R⁴: Halogen, CF₃ oder Nitro;
- R⁵: Wasserstoff oder Halogen bedeuten, sowie Verbindung 8-Chlor-6-(2-fluorphenyl)-1-morpholin-4ylmethyl-4H-imidazo[1,2a] [1,4]-benzodiazepin-2-methanol-hydrochlorid(1:1)
sowie pharmazeutisch annehmbare Salze von allgemeiner Formel I.

Der Ausdruck "nieder" bezeichnet Reste oder Verbindungen mit höchstents 7, vorzugsweise höchstens 4 Kohlenstoffatomen.

Diese Verbindungen und Salze sind neu und besitzen wertvolle pharmakodynamische Eigenschaften. Sie eignen sich daher für therapeutische Zwecke, insbesondere für anxiolytische und/oder anti-konvulsive und/oder muskelrelaxierende und/oder sedativ-hypnotische Zwecke. Ein besonderer Vorteil der neuen Verbindungen der allgemeinen Formel I ist die gute Wasserlöslichkeit ihrer Salze sowie eine kurze Wirkungsdauer für den oben genannten Verwendungszweck.

Die Patentschrift US 4,902,794 beschreibt 1-substituierte-6-phenyl-4H-s-triazolo[4,3-a]benzodiazepine, die als Sedativum und muskelrelaxierendes Mittel verwendet werden können.

Die Europäische Patentanmeldung 0 139 460 beschreibt die Verwendung von 1,4-substituierten 8-Chloro-6-phenyl-4-H-s-triazolo[4,3-a]benzodiazepinen für die Behandlung von Schizophrenie.

Die Patentschrift US 4,280,957 beschreibt Imidazobenzodiazepine, die anxiolytische, anti-konvulsive und muskelrelaxierende Eigenschaften aufweisen.

Gegenstand der vorliegenden Erfindung sind die erwähnten Verbindungen der Formel I und Salze davon als solche und als therapeutische Wirkstoffe, deren Herstellung und deren Verwendung für therapeutische Zwecke bzw. zur Herstellung entsprechender Arzneimittel sowie Arzneimittel, enthaltend eine Verbindung der Formel I oder ein Salz davon, und die Herstellung solcher Arzneimittel.

Der Ausdruck "Alkyl" bezeichnet geradkettige oder verzweigte gesättigte Kohlenwasserstoffreste, wie Methyl, Ethyl, n-Propyl, iso-Propyl, n-Butyl, sek.-Butyl, iso-Butyl und tert.-Butyl. Der Ausdruck "Alkenyl" bezeichnet geradkettige oder verzweigte Kohlenwasserstoffreste, welche eine C-C-Doppelbindung enthalten, wie Allyl, But-2-enyl, 3-Methyl-but-2-enyl und dgl. Der Ausdruck "Halogen" umfasst Fluor, Chlor, Brom und Jod. Der Ausdruck "Amino" bezeichnet solche Reste wie NH₂, NH-nieder-Alkyl, N-Di-nieder-alkyl, NH-nieder-Alkenyl, Morpholino, Piperidino, Pyrrolidin-1-yl, Methylpiperazin-1-yl und dergleichen. Der Ausdruck "Alkanoyl" bezeichnet Reste wie Acetyl, Propionyl und dergleichen.

Bedeutet A in Formel I einen Rest der Formel A¹, sind solche Verbindungen bevorzugt, worin R¹ Wasserstoff, R² o-Fluor- oder o-Cl-Phenyl, R³ Wasserstoff oder Allylaminomethyl und R⁴ Jod oder Chlor bedeuten.

Beispiele hierfür sind die folgenden Verbindungen:
8-Chlor-6-(2-fluorphenyl)-4H-imidazo [1,2-a]benzodiazepin-2-methanol;
1-Allylaminomethyl-8-chlor-6-(2-fluorphenyl)-4H-imidazo [1,2-a]-[1,4]benzodiazepin-2-methanol;
8-Chlor-6-(2-chlorphenyl)-4H-imidazo[1,2-a][1,4]benzodiazepin-2-methanol;
8-Jod-6-(2-fluorphenyl)-4H-imidazo[1,2-a][1,4]benzodiazepin-2-methanol.

Bedeutet A in Formel I einen Rest der Formel A², sind solche Verbindungen bevorzugt, worin R¹ Wasserstoff oder Carbonylmethyl, R² o-Fluorphenyl, R³ Wasserstoff und R⁵ Chlor bedeuten.

Beispiele hierfür sind die folgenden Verbindungen:
2-Chlor-4-(2-fluorphenyl)-8,9-dihydro-6H-imidazo[1,2-a]thieno[3,2-f][1,4]diazepin-8-methanol;
Essigsäure-[2-chlor-4-(2-fluorphenyl)-8,9-dihydro-6H-imidazo-[1,2-a]thieno[3,2-f]diazepin-8-yl]methylester.

Die eingangs erwähnten Verbindungen der allgemeinen Formel I und deren pharmazeutisch annehmbaren Säureadditionssalze können erfindungsgemäss dadurch hergestellt werden, dass man
a) Verbindungen der allgemeinen Formeln 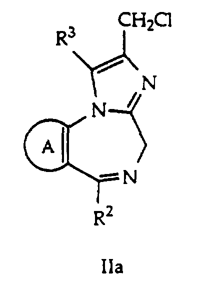 mit einem basischen Reagens zu Verbindungen der allgemeinen Formel 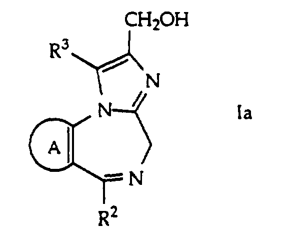 worin A, R² und R³ die obige Bedeutung haben, umsetzt, oder
b) Verbindungen der allgemeinen Formel Ia oder IIa zu Verbindungen der allgemeinen Formel 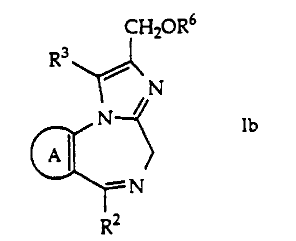 worin A, R² und R³ die obige Bedeutung haben, und R⁶ nieder-Alkanoyl bedeutet,
   umsetzt, oder
c) Verbindungen der allgemeinen Formel Ib zu Verbindungen der allgemeinen Formel Ia hydrolysiert, oder
d) Verbindungen der allgemeinen Formel 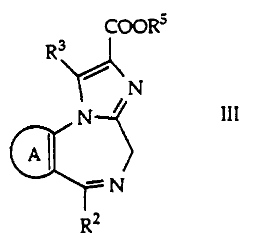 worin A, R² und R³ die obige Bedeutung haben und R⁵ nieder-Alkyl bedeutet,
   zu Verbindungen der allgemeinen Formel 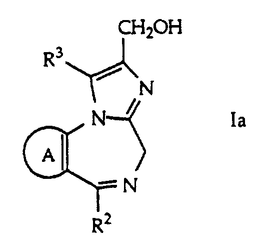 reduziert, oder
e) aus einer Verbindung der allgemeinen Formel 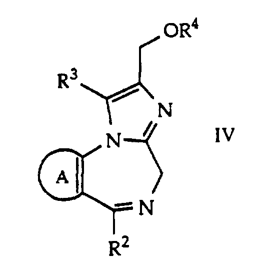 worin A, R² und R³ die obige Bedeutung haben, und R⁴ eine Schutzgruppe bedeutet,
   die Schutzgruppe abspaltet, oder
f) Verbindungen der allgemeinen Formel 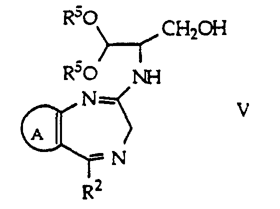 worin A und R² die obige Bedeutung haben und R⁵ nieder-Alkyl bedeutet,
   im sauren Medium zu Verbindungen der allgemeinen Formel Ia, in welcher R³ Wasserstoff bedeutet, cyclisiert, und
g) gegebenenfalls eine Verbindung der allgemeinen Formel I in ein pharmazeutisch anwendbares Säureadditionssalz überführt.

Nach Verfahrensvariante a) können Verbindungen der allgemeinen Formel Ia hergestellt werden, indem man Verbindungen der Formeln IIa mit einem basischen Reagens behandelt. Dabei geht man zweckmässigerweise folgendermassen vor: Eine Verbindung der Formel IIa wird unter Schutzgasatmosphäre mehrere Stunden in einer Alkalihydroxidlösung, vorzugsweise in Natriumhydroxid, gerührt oder mit Natriumhydrogencarbonat behandelt und anschliessend nach bekannten Methoden aufgearbeitet.

Gemäss Verfahrensvariante b) erhält man Verbindungen der allgemeinen Formel Ib, ausgehend von Verbindungen der Formel Ia, indem man diese Verbindungen zweckmässigerweise mit Essigsäureanhydrid o.dgl. in Anwesenheit einer starken Säure, beispielsweise Perchlorsäure, umsetzt, oder, ausgehend von einer Verbindung der Formel IIa, diese mit Natriumacetat o.dgl. umsetzt. Diese Methode ist jedem Fachmann geläufig.

Nach Verfahrensvariante c) können die Verbindungen der allgemeinen Formel Ib zu Verbindungen der allgemeinen Formel Ia hydrolysiert werden. Das erfolgt zweckmässigerweise mit einer methanolischen Natriummethanolat-Lösung.

Gemäss Verfahrensvariante d) erhält man Verbindungen der Formel Ia. Das erfolgt durch Reduktion einer Verbindung der allgemeinen Formel III mit einem geeigneten Reduktionsmittel, beispielsweise mit einer Lithiumaluminiumhydrid-Lösung. Dabei geht man zweckmässigerweise folgendermassen vor: Das Reduktionsmittel wird in einem geeigneten organischen Lösungsmittel, beispielsweise Tetrahydrofuran gelöst, auf ca. -70 bis -80°C abgekühlt und mit einer Lösung einer Verbindung der allgemeinen Formel III versetzt. Nach einer Reaktionszeit von ca. 1 Stunde wird langsam auf 0°C erwärmt und anschliessend nach üblichen Methoden aufgearbeitet.

Gemäss Verfahrensvariante e) erhält man Verbindungen der allgemeinen Formel Ia. Geeignete Schutzgruppen und Methoden zu deren Abspaltung sind jedem Fachmann geläufig, wobei natürlich nur solche Schutzgruppen verwendet werden können, die sich durch Methoden abspalten lassen, unter deren Bedingungen andere Strukturelemente in den Verbindungen nicht in Mitleidenschaft gezogen werden. Zur Herstellung von Verbindungen der allgemeinen Formel I, worin R¹ Wasserstoff und R³ Aminomethyl bedeutet und R² die oben genannte Bedeutung hat, kann man zweckmässigerweise so vorgehen, dass man eine Verbindung der allgemeinen Formel IVb, die eine Schutzgruppe trägt, in einem Verfahrensschritt zu Verbindungen der allgemeinen Formel Ia umsetzen kann, indem man die reaktiven Verbindungen der Formel IVb mit einem entsprechenden Amin behandelt und durch Arbeiten im sauren Bereich gleichzeitig die Schutzgruppe abspalten kann. Als Amine sind die folgenden besonders bevorzugt: Diethylamin, Morpholin, Methylamin, Allylamin und dergleichen.

Gemäss Verfahrensvariante g) können die Verbindungen der Formel I in pharmazeutisch annehmbare Säureadditionssalze übergeführt werden. Es kommen dabei sowohl Salze mit anorganischen als auch Salze mit organischen Säuren in Betracht. Beispiele derartiger Salze sind die Hydrochloride, Hydrobromide, Sulfate, Nitrate, Citrate, Acetate, Maleate, Succinate, Methansulfonate, p-Toluolsulfonate und dergleichen. Diese Salze können nach an sich bekannten und jedem Fachmann geläufigen Methoden hergestellt werden.

Die Ausgangsprodukte der obigen Formeln IIa, III, IVb und V können gemäss den nachfolgenden Schemata 1 oder 2 hergestellt werden.

In Schema 1 und 2 haben A und R² die oben genannte Bedeutung, R⁵ bedeutet nieder-Alkyl und R⁶ bedeutet Halogen.

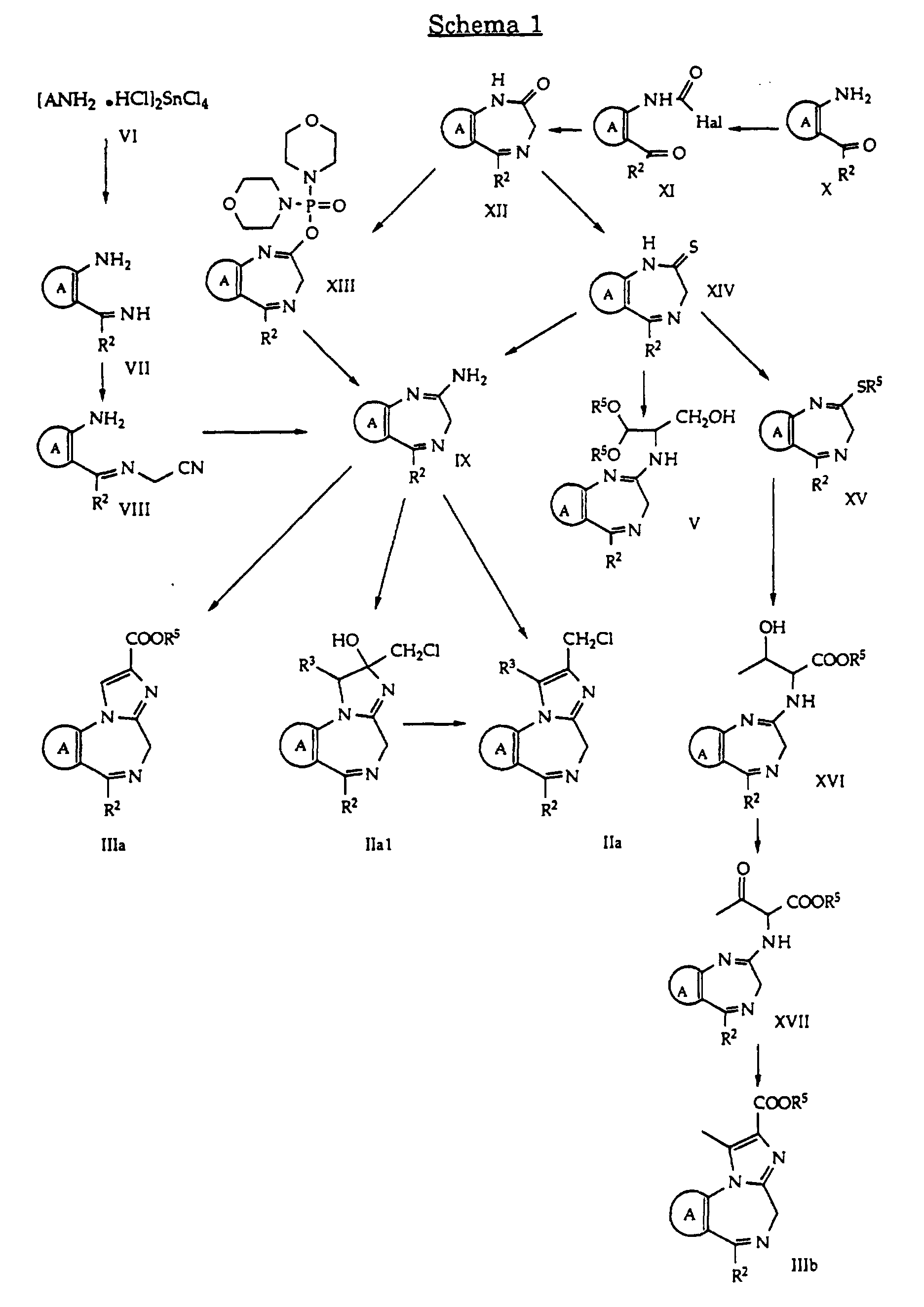

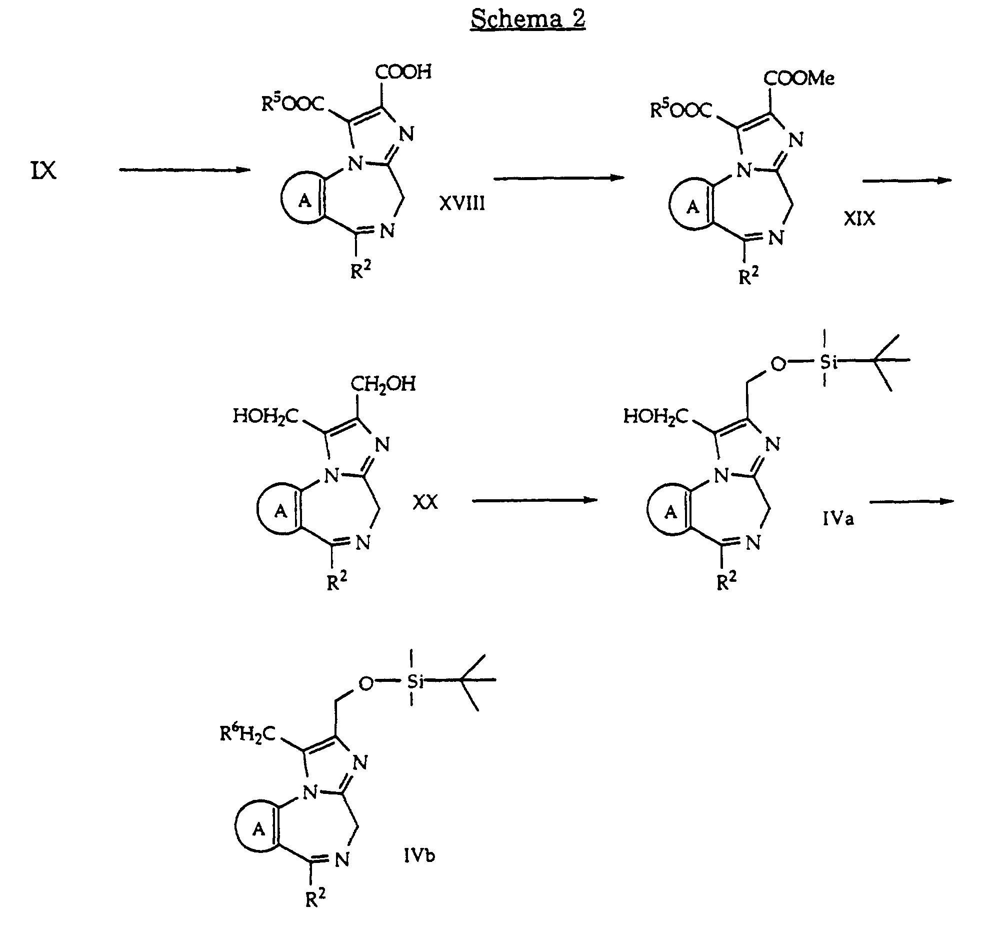

Eine zentrale Bedeutung für die Herstellung der erfindungsgemässen Verbindungen der allgemeinen Formel I haben die Verbindungen der allgemeinen Formeln IX und XIV. Diese können gemäss Schema 1 auf unterschiedlichem Wege hergestellt werden. Diese Möglichkeiten sind ausführlich in den Synthesebeispielen 1a, 1b, 1c, 3a, 3b, 4, 14a, 14b, 15a, 16a, 17a, 18a, 19a, 19b, 20a, 20b, 20c und 20d beschrieben. Die Verbindungen der allgemeinen Formeln IX und XIV sind bekannte Verbindungen und sind beschrieben in J. Med. Chem., 1971, 14, 1978-1081; Am. Chem. Soc., 1953, 75, 6244; Yakugaku Zasshi, 1973, 93, 1253-1262; J. Chem. Res. Synop., 1980, 400; J. Med. Chem., 1991, 34, 1209; J. Med. Chem., 1980, 23, 392-402; DE-OS 2.233.483.

Die Herstellung einer Verbindung der Formel IIIa, ausgehend von einer Verbindung der allgemeinen Formel IX soll anhand eines Beispieles, worin A und R² die obige Bedeutung haben und Alk Ethyl bedeutet, näher beschrieben werden.

Ein entsprechendes Diazepin der allgemeinen Formel IX wird nacheinander mit N-Ethyldiisopropylamin und Brombrenztraubensäure-ethylester (Bsp. 18), gelöst in Tetrahydrofuran, unter Erwärmen mehrere Minuten behandelt, abekühlt, mit Diethylether versetzt und das entstandene N-Ethyldiisopropylamin-hydrobromid abfiltriert. Das Filtrat wurde anschliessend nach bekannten Methoden in die Zielverbindungen der Formel IIIa überführt.

Verbindungen der allgemeinen Formel IIa erhält man gemäss Schema 1 durch Umsetzung einer Verbindung der allgemeinen Formel IX mit 1,3-Dichloraceton in Anwesenheit von Natriumbicarbonat. Zweckmässigerweise erfolgt diese Umsetzung in einem inerten Lösungsmittel, wie z.B. Dioxan unter Schutzgasatmosphäre bei Raumtemperatur.

Verbindungen der allgemeinen Formel IIb erhält man auf ähnliche Weise. Man setzt eine Verbindung der allgemeinen Formel IX mit 1,3-Dichloraceton in Anwesenheit von wasserfreiem Kaliumcarbonat oder Natriumhydrogencarbonat in Dioxan um und arbeitet anschliessend nach bekannten Methoden auf.

Ausgehend von Verbindungen der allgemeinen Formel XIV erhält man Verbindungen der allgemeinen Formel V, indem man ein Diazepin der allgemeinen Formel XIV mit 3,3-Diethoxy-1-hydroxy-2-propylamin, gelöst in einem Alkohol, beispielsweise in Butanol, umsetzt. Das erfolgt zweckmässigerweise innert mehrerer Stunden unter Erwärmen.

Ebenfalls in Schema 1 dargestellt ist eine Verfahrensvariante, die über mehrere Stufen zur Herstellung von Verbindungen der allgemeinen Formel IIIb führt.

Ausgehend von einer Verbindung der allgemeinen Formel XIV erhält man Verbindungen der allgemeinen Formel XV durch Alkylierung. Ein geeignetes Alkylierungsmittel ist Methyljodid; aber auch andere Alkylierungsmittel sind möglich.

Zweckmässigerweise geht man dabei so vor, dass eine Verbindung der allgemeinen Formel XIV in Anwesenheit von Kaliumcarbonat und dem Alkylierungsmittel in einem geeigneten Lösungsmittel, z.B. Aceton gelöst, unter Rühren am Rückfluss gekocht wird. Nach erfolgter Aufarbeitung wird die Verbindung der Formel XV mit L-Threonin-alkylester-hydrochlorid, gelöst in Pyridin, zu Verbindungen der Formel XVI umgesetzt. Man erhält diese Verbindung als Diastereomerengemisch, das ohne Reinigung zu Verbindungen der allgemeinen Formel XVII umgesetzt wird. Dazu lässt man diese Verbindung, gelöst in Dichlormethan, zu einer Lösung zutropfen, die folgendermassen zubereitet wird: Man tropft eine Lösung, bestehend aus Trifluoressigsäureanhydrid in Dichlormethan zu einer gekühlten Lösung aus Dimethylsulfoxid und Dichlormethan und versetzt dieses Lösungsgemisch anschliessend mit einer Verbindung der allgemeinen Formel XVI. Die erhaltenen Verbindungen der allgemeinen Formel XVII werden anschliessend zu Verbindungen der allgemeinen Formel IIIb cyclisiert. Das geschieht zweckmässigerweise in Dimethylformamid in Anwesenheit von p-Toluolsulfonsäure-monohydrat unter Erwärmung.

Das Schema 2 zeigt die Herstellung von Verbindungen der allgemeinen Formel IVb, ausgehend von Verbindungen der allgemeinen Formel IX. Dabei geht man zweckmässigerweise so vor: Eine Verbindung der allgemeinen Formel IX wird in Anwesenheit von Dihydroxyfumarsäure-dimethylester und Triphenylphosphin, gelöst in Tetrahydrofuran, unter Kühlung mit Azodicarbonsäure-diethylester, gelöst in Tetrahydrofuran, umgesetzt. Nach der Aufarbeitung wird die entstandene Verbindung der allgemeinen Formel XVIII zweckmässigerweise in Tetrahydrofuran gelöst und in Anwesenheit von Dimethylformamid mit Oxalylchlorid behandelt. Das so entstandene Säurechlorid wird durch Methanolyse in XIX überführt. XIX wird anschliessend zu Verbindungen der allgemeinen Formel XX reduziert. Das geschieht zweckmässigerweise nach allgemein bekannten Methoden. Als Reduktionsmittel eignet sich Lithiumaluminiumhydrid in Tetrahydrofuran besonders gut. Nach erfolgter Reduktion wird die OH-Gruppe geschützt, an der keine weitere Umsetzung mehr erfolgen soll. Zweckmässigerweise wird dazu das entsprechende Diazepin der allgemeinen Formel XX in Anwesenheit von Imidazol, gelöst in Dimethylformamid, mit tert-Butyldimethylsilylchlorid bei Raumtemperatur umgesetzt. Die entstandene Verbindung der allgemeinen Formel IVa kann anschliessend für weitere Umsetzungen nach allgemein bekannten Methoden mit geeignetem Aktivierungsmittel, beispielsweise Tetrabrommethan und Triphenylphosphin, in reaktive Verbindungen der allgemeinen Formel IVb überführt werden.

Eine nähere Beschreibung der in Schema 2 hergestellten Verbindungen erfolgt in den Synthesebeispielen 6a, 6b, 6c, 6d, 6e, 7, 8 und 9.

Wie eingangs erwähnt sind die Verbindungen der Formel I neu. Sie besitzen wertvolle pharmakodynamische Eigenschaften und weisen nur eine geringe Toxizität auf. Sie besitzen als gemeinsames Merkmal eine ausgeprägte Affinität zu den zentralen Benzodiazepin-Rezeptoren und haben aufgrund ihrer agonistischen Wirkung an diesen Rezeptoren ausgeprägte anxiolytische, antikonvulsive, muskelrelaxierende und sedativ-hypnotische Eigenschaften. Sie bilden sehr gut wasserlösliche Säureadditionssalze und eignen sich daher vorzüglich zur Herstellung von wässerigen Injektionslösungen.

Die Affinität von Verbindungen der allgemeinen Formel I zu den zentralen Benzodiazepin-Rezeptoren wurde in vitro nach der in Nature 294, 763-765 (1981) und J. Neurochemistry 37, 714-722 (1981) beschriebenen Methode festgestellt. Nach dieser Methode ermittelt man die Hemmung der Bindung von tritiiertem Flumazenil an die spezifischen Benzodiazepin-Rezeptoren im Ratten-Cortex durch die jeweiligen Testsubstanzen. Man bezeichnet als IC₅₀ ("50% inhibiting concentration") diejenige Konzentration der jeweiligen Testsubstanz, welche eine 50-proz. Hemmung der spezifischen Bindung des tritiierten Flumazenils an die spezifisichen Benzodiazepin-Rezeptoren im Ratten-Cortex bewirkt.

Die sedativ/muskelrelaxierenden Eigenschaften der erfindungsgemässen Verbindungen der Formel II können beispielsweise im Rotierstab-Test erfasst werden. Für diesen Test werden Mäuse von 19-21 g Gewicht verwendet. Bis 1 Stunde vor Beginn des Versuches haben sie freien Zugang zu Futter und Trinkwasser. Mindestens 30 Minuten vor dem Versuch werden sie in das Versuchslabor gebracht. Beim Rotierstab-Test werden die Tiere auf einen waagrecht angeordneten, glatten Metallstab von 3 cm Durchmesser, der sich mit 2 Umdrehungen pro Minute dreht, aufgesetzt. Zunächst lässt man den Tieren 30 Sekunden Gelegenheit, sich mit der Test-Situation vertraut zu machen. Anschliessend werden diejenigen Tiere ausgewählt, denen es gelingt, mindestens 1 Minute lang auf dem Stab zu verbleiben. Diesen Tieren werden dann die Versuchspräparate in verschiedenen Dosen intravenös verabreicht. Zu verschiedenen Zeitpunkten wird dann bestimmt, ob sich die Tiere während einer Mindestzeit auf dem Stab halten können (Mindestzeit: 10 Sekunden, ab 5 Minuten nach Verabreichung: 1 Minute). Es wird diejenige Dosis ermittelt, bei welcher sich 50% der Tiere auf dem Stab zu halten vermögen (ED₅₀).

In der nachfolgenden Tabelle werden die Resultate dargestellt, welche mit repräsentativen Vertretern der durch die allgemeinen Formel I definierten Verbindungsklasse in den vorstehend geschilderten Versuchen erhalten worden sind.

**Tabelle**

| | | Rotierstab, Test, ED₅₀ in mg/kg; i.v., ermittelt zu folgenden Zeitpunkten nach Verabreichung | | | | | | | |
|---|---|---|---|---|---|---|---|---|---|
| | | Mindestzeit: 10 sec | | | | Mindestzeit: 60 sec | | | |
| Verbindung Beispiel | Affinität zu Benzodiazepin- Rezeptoren IC₅₀[nMol] | 15 sec | 30 sec | 60 sec | 2 min | 5 min | 15 min | 30 min | 60 min |
| A 1f, 2 | 2,6 | 0,5 | 0,7 | 0,6 | 1,2 | 2,3 | 5,3 | ≥10 | >10 |
| B 1g | 6,0 | 0,6 | 0,7 | 1,2 | 1,0 | ≥10 | ≥10 | ≥10 | >10 |
| C 4b, 10b | 1,7 | 0,1 | 0,3 | 0,3 | ≥10 | 0,8 | 0,4 | 1,1 | ≥10 |
| D 9 | 5,6 | 2,5 | 3,0 | 3,2 | 3,2 | 2,1 | 1,8 | ≥10 | >10 |
| E 11 | 1,2 | ≤0,32 | 0,4 | 0,5 | 3,2 | 0,9 | 0,5 | ≥3,2 | ≥3,2 |
| F 13 | 12,0 | 1,0 | 1,1 | 1,1 | >3,2 | 1,0 | 0,9 | 1,1 | 1,8 |
| G 14c | 2,3 | 0,2 | 0,2 | 0,3 | 0,3 | 0,1 | 0,2 | 0,2 | ≥1 |
| H 17b | 2,07 | 0,1 | 0,06 | 0,09 | 0,1 | 0,1 | 0,1 | ≥1 | >1 |
| I 18c | 40 | 1,5 | 2,0 | 3,0 | >10 | 1,4 | 1,8 | 3,0 | ≥10 |
| Dabei bedeuten: | | | | | | | | | |
| A 2-Chlor-4-(2-fluorphenyl)-8,9-dihydro-6H-imidazo[1,2-a]thieno-[3,2-f][1,4]diazepin-8-methanol | | | | | | | | | |
| B Essigsäure-[2-chlor-4-(2-fluorphenyl)-8,9-dihydro-6H-imidazo-[1,2-a]thieno[3,2-f]diazepin-8-yl]methylester | | | | | | | | | |
| C 8-Chlor-6-(2-fluorphenyl)-4H-imidazo[1,2-a]benzodiazepin-2-methanol | | | | | | | | | |
| D 1-Allylaminomethyl-8-chlor-6-(2-fluorphenyl)-4H-imidazo[1,2-a][1,4]benzodiazepin-2-methanol | | | | | | | | | |
| E 8-Chlor-6-(2-chlorphenyl)-4H-imidazo[1,2-a][1,4]benzodiazepin-2-methanol | | | | | | | | | |
| F 8-Trifluormethyl-6-phenyl-4H-imidazo[1,2-a][1,4]benzodiazepin-2-methanol | | | | | | | | | |
| G 6-(2-Fluorphenyl)-8-nitro-4H-imidazo[1,2-a][1,4]benzodiazepin-2-methanol | | | | | | | | | |
| H 6-(2-Fluorphenyl)-8-jod-4H-imidazo[1,2-a][1,4]benzodiazepin-2-methanol | | | | | | | | | |
| I 8-Brom-6-(2-pyridyl)-4H-imidazo[1,2-a][1,4]benzodiazepin-2-methanol | | | | | | | | | |

Aus der obigen Tabelle ist ersichtlich, dass Verbindungen A bis I eine sehr rasch eintretende und nur relativ kurze Zeit anhaltende sedative Wirkung entfalten.

Die Verbindungen der Formel I können aufgrund ihrer agonistischen Wirkung auf die Benzodiazepin-Rezeptoren als Sedativa/Hypnotika, Antikonvulsiva, Muskelrelaxantien und Anxiolytika verwendet werden. Sie eignen sich beispielsweise als rasch aber kurz wirksame Hypnotika für perorale Verabreichung, insbesondere aber - in Form wässeriger Lösungen ihrer Säureadditionssalze - als injizierbare Kurzhypnotika für Prämedikation, Sedation sowie Narkoseeinleitung und Aufrechterhaltung der Narkose; bevorzugte Anwendungsmöglichkeiten sind dabei Prämedikation vor der Narkoseeinleitung, Basalsedation vor diagnostischen oder chirurgischen Eingriffen mit oder ohne Lokalanästhesie, Langzeitsedation auf der Intensivpflegestation, Verwendung als Induktionsmittel der Inhalationsnarkose oder als schlafinduzierende Komponente der Kombinationsnarkose (einschliesslich der totalen intravenösen Anästhesie) usw.

Die Verbindungen der Formel I und pharmazeutisch annehmbare Säureadditionssalze davon können als Heilmittel, z.B. in Form pharmazeutischer Präparate, Verwendung finden. Die pharmazeutischen Präparate können oral, z.B. in Form von Tabletten, Lacktabletten, Dragées, Hart- und Weichgelatinekapseln, Lösungen, Emulsionen oder Suspensionen, verabreicht werden. Die Verabreichung kann aber auch rektal, z.B. in Form von Suppositorien, oder parenteral, z.B. in Form von Injektionslösungen, erfolgen.

Zur Herstellung von pharmazeutischen Präparaten können die Verbindungen der Formel I und pharmazeutisch annehmbare Säureadditionssalze davon mit pharmazeutisch inerten, anorganischen oder organischen Trägern verarbeitet werden. Als solche Träger kann man für Tabletten, Lacktabletten, Dragées und Hartgelatinekapseln beispielsweise Lactose, Maisstärke oder Derivate davon, Talk, Stearinsäure oder deren Salze und dergleichen verwenden. Für Weichgelatinekapseln eignen sich als Träger beispielsweise pflanzliche Oele, Wachse, Fette, halbfeste und flüssige Polyole und dergleichen; je nach Beschaffenheit des Wirkstoffes sind jedoch bei Weichgelatinekapseln überhaupt keine Träger erforderlich. Zur Herstellung von Lösungen und Sirupen eignen sich Träger beispielsweise Wasser, Polyole, Saccharose, Invertzucker, Glukose und dergleichen. Für wässerige Injektionslösungen wasserlöslicher Säureadditionssalze von Verbindungen der Formel I sind Hilfsstoffe, wie Alkohole, Polyole, Glyzerin, pflanzliche Oele und dergleichen verwendbar aber in der Regel nicht notwendig. Für Suppositorien eignen sich als Träger beispielsweise natürliche oder gehärtete Oele, Wachse, Fette, halbflüssige oder flüssige Polyole und dergleichen.

Die pharmazeutischen Präparate können daneben noch Konservierungsmittel, Lösungsvermittler, Stabilisierungsmittel, Netzmittel, Emulgiermittel, Süssmittel, Färbemittel, Aromatisierungsmittel, Salze zur Veränderung des osmotischen Druckes, Puffer, Ueberzugsmittel oder Antioxidantien enthalten. Sie können auch noch andere therapeutisch wertvolle Stoffe enthalten.

Wie eingangs erwähnt, sind Arzneimittel, enthaltend eine Verbindung der Formel I oder ein pharmazeutisch annehmbares Säureadditionssalz davon und ein therapeutisch inertes Excipiens. ebenfalls Gegenstand der vorliegenden Erfindung, weiterhin auch ein Verfahren zur Herstellung solcher Arzneimittel, welches dadurch
gekennzeichnet ist, dass man eine oder mehrere Verbindungen der Formel I oder pharmazeutisch annehmbare Säureadditionssalze davon und gegebenenfalls einen oder mehrere andere therapeutisch wertvolle Stoffe zusammen mit einem der mehreren therapeutisch inerten Trägern in eine galenische Darreichungsform bringt.

Wie eingangs erwähnt, können die Verbindungen der Formel I und pharmazeutisch annehmbare Säureadditionssalze davon erfindungsgemäss für therapeutische Zwecke verwendet werden und zwar insbesondere für anxiolytische und/oder antikonvulsive und/oder muskelrelaxierende und/oder sedativ-hypnotische Zwecke. Die Dosierung kann innerhalb weiter Grenzen variieren und ist natürlich in jedem einzelnen Fall den individuellen Gegebenheiten anzupassen. Im allgemeinen dürfte bei intravenöser Verabreichung eine Tagesdosis von etwa 1 mg bis 1000 mg angemessen sein.

Ebenfalls Gegenstand der Erfindung ist schliesslich, wie eingangs erwähnt, die Verwendung von Verbindungen der Formel I und von pharmazeutisch anwendbaren Säureadditionssalzen davon für die Herstellung von Arzneimitteln, insbesondere von anxiolytischen und/oder antikonvulsiven und/oder muskelrelaxierenden und/oder sedativhypnotischen Arzneimitteln.

Die nachfolgenden Beispiele sollen die vorliegende Erfindung näher erläutern.

### Beispiel 1

### Herstellung von 2-Chlor-4-(2-fluor-phenyl)-8,9-dihydro-6H-imidazo[1,2-a]thieno[3,2-f][1,4]diazepin-8-methanol

a) Eine Suspension von 100 g Dihydrogen-hexachlor-stannat-bis(thiophen-2-ylamin), 50.7 g 2-Fluorbenzonitril und 54.7 g Aluminiumchlorid (wasserfrei) in 380 ml 1,2-Dichlorethan und 750 ml Toluol wurde bei Raumtemperatur unter Stickstoffbegasung 0.5 h gerührt und mit 380 ml Bortrichlorid (1 M in Xylol) vorsichtig versetzt. Die Reaktionsmischung wurde 1 h bei 65°C und 5 h bei 110°C gerührt. Die Lösung wurde auf 1300 ml Ammoniak (16%-ig) in Methanol, 1000 ml Dichlormethan und Eis gegossen, anschliessend durch Hyflo filtriert. Die organische Phase wurde mit gesättigter Natriumchlorid-Lösung gewaschen, mit Natriumsulfat getrocknet, filtriert und im Vakuum zur Trockene eingedampft. Der Rückstand, 73 g rohes 3-[(2-Fluor-phenyl)-imino-methyl]-thiophen-2-ylamin) wurde für die untenstehend beschriebene Stufe ohne weitere Reinigung als Ausgangsprodukt verwendet.
b) Eine Lösung von 73 g 3-[(2-Fluor-phenyl)-imino-methyl]-thiophen-2-ylamin und 48 g Aminoacetonitril-hydrochlorid in 1150 ml Methanol wurde 1 h bei 75°C unter Argon gerührt. Die Reaktionsmischung wurde mit Wasser/Dichlormethan extrahiert. Die Dichlormethan-Extrakte wurden über Natriumsulfat getrocknet, filtriert und im Vakuum zur Trockene eingedampft. Der Rückstand (70 g) [(2-Aminothiophen-3-yl)-(2-fluor-phenyl)-methylenamino]acetonitril wurde für die untenstehend beschriebene Stufe als Ausgangsprodukt verwendet.
c) Eine Lösung von 70 g [(2-Amino-thiophen-3-yl)-(2-fluor-phenyl)-methylenamino] acetonitril in 980 ml Essigsäure wurde während 1.5 h bei 65°C unter Argon gerührt. Nach Abkühlen wurde das Lösungsmittel im Vakuum entfernt, der Rückstand wurde in Dichlormethan gelöst und mit Natriumbicarbonat-Lösung unter Eiskühlung neutralisiert und extrahiert. Die Dichlormethan-Extrakte wurden mit Natriumsulfat getrocknet, filtriert und im Vakuum zur Trockene eingedampft. Nach Kristallisation aus Dichlormethan erhielt man 17.1 g 5-(2-Fluor-phenyl)-3H-thieno[2,3-e][1,4]diazepin-2-ylamin vom Smp. 219-220°C (Zers.).
d) 19 g 5-(2-Fluor-phenyl)-3H-thieno[2,3-e][1,4]diazepin-2-ylamin wurden in 730 ml 5%-igem Methanol in Dichlormethan gelöst, unter gutem Rühren mit 327 g einer 2.8%-igen Lösung von Chlorgas in 5% Methanol in Dichlormethan versetzt und 5 Minuten unter Argon bei Raumtemperatur gerührt. Danach wurde die Reaktionsmischung unter Eiskühlung auf 600 ml 0.2 N Natriumhydroxid-Lösung gegossen und extrahiert. Die Dichlormethan-Extrakte wurden mit Natriumsulfat getrocknet, filtriert, und das Lösungsmittel im Vakuum eingedampft. Nach zweimaligem Aufkochen des Rückstandes im 5% Methanol in Dichlormethan und Filtration erhielt man 7.4 g 7-Chlor-5-(2-fluor-phenyl)-3H-thieno[2,3-e][1,4]diazepin-2-ylamin vom Smp. 215-217°C (Zers.).
e) Eine Suspension von 9.5 g 7-Chlor-5-(2-fluor-phenyl)-3H-thieno-[2,3-e][1,4]diazepin-2-ylamin, 4.8 g 1,3-Dichloraceton, 31.5 g Natriumbicarbonat in 220 ml Dioxan wurde während 48 h unter Argon bei Raumtemperatur gerührt. Danach wurde die Temperatur auf 80°C erhöht und die Reaktionsmischung während weiteren 24 h unter Argon gerührt. Nach Abkühlen der Suspension auf 60°C wurde das rohe (RS)-2-Chlor-8-chlormethyl-4-(2-fluor-phenyl)-8,9-dihydro-6H-imidazo[1,2-a]thieno[3,2-f][1,4]diazepin-8-ol ohne Aufarbeitung und Reinigung für die untenstehend beschriebene Stufe verwendet.
f) Zu einer Suspension des gemäss Absatz e) erhaltenen Reaktionsgemisches ((RS)-2-Chlor-8-chlormethyl-4-(2-fluor-phenyl)-8,9-dihydro-6H-imidazo[1,2-a]thieno[3,2-f][1,4]diazepin-8-ol wurden bei 60°C 37.2 g 2.5 N Natriumhydroxid-Lösung gegeben und 21 h unter Argon gerührt, anschliessend wurden noch in zweistündigen Abständen zweimal 18.7 g 2.5 N Natriumhydroxid-Lösung zugegeben und 2 h nachgerührt. Die Suspension wurde mit Dichlormethan gesättigter Natriumchlorid-Lösung in Gegenwart von wenig Methanol extrahiert. Die Dichlormethan-Extrakte wurden mit Natriumsulfat getrocknet, filtriert und im Vakuum zur Trockene eingedampft. Nach Kristallisation des Rückstandes aus 5% Methanol in Dichlormethan erhielt man 3.3 g des Ausgangsproduktes der Stufe e) zurück. Die Mutterlaugen wurden im Vakuum zur Trockene eingedampft und der Rückstand an 800 g Kieselgel chromatographiert. Mit 5% Methanol in Dichlormethan eluierte man 1.5 g 2-Chlor-4-(2-fluor-phenyl)-8,9-dihydro-6H-imidazo-[1,2-a]thieno[3,2-f][1,4]diazepin-8-methanol, welches ohne weitere Reinigung als Ausgangsprodukt für die untenstehend beschriebene Stufe verwendet wurde.

### Herstellung von Essigsäure-[2-chlor-4-(2-fluor-phenyl)-8,9-dihydro-6H-imidazo[1,2-a]thieno[3,2-f][1,4]diazepin-8-yl-methyl-hydrochlorid

g) Zu einer Suspension von 1.5 g 2-Chlor-4-(2-fluor-phenyl)-8,9-dihydro-6H-imidazo[1,2-a]thieno[3,2-f][1,4]diazepin-8-ylmethanol in 31 ml Essigsäureanhydrid wurden 31 Tropfen Perchlorsäure (70%-ig) gegeben, bis das Ausgangsprodukt ganz in Lösung war. Nach 20 min Rühren bei Raumtemperatur wurde die Lösung auf Natriumcarbonat/Eis/Dichlormethan gegossen und während 1 h bei Raumtemperatur gerührt. Nach Extraktion wurden die Dichlormethan-Extrakte mit Natriumsulfat getrocknet, filtriert und im Vakuum zur Trockene eingedampft. Der Rückstand wurde 2 h im Hochvakuum getrocknet, in 30 ml Aceton gelöst, mit 52 g 1.6%-igem Salzsäuregas in Diethylether versetzt, im Vakuum (223 mbar) eingedampft und aus Aceton kristallisiert. Nach Kristallisaton aus Aceton erhielt man 1 g Essigsäure-[2-chlor-4-(2-fluor-phenyl)-8,9-dihydro-6H-imidazo[1,2-a]-thieno[3,2-f][1,4]diazepin-8-yl-methyl-hydrochlorid vom Smp. 126-129°C (Zers., stark hygroskopisch).

### Beispiel 2

### Herstellung von 2-Chlor-4-(2-fluor-phenyl)-8,9-dihydro-6H-imidazo-[1,2-a]thieno[3,2-f][1,4]diazepin-8-methanol-hydrochlorid

Eine Lösung von 450 mg Essigsäure-(2-Chlor-4-(2-fluor-phenyl)-8,9-dihydro-6H-imidazo[1,2-a]diazepin-8-ylmethyl)ester in 15 ml Methanol wurde mit 3 ml 3.5%-iger Natriummethanolat-Lösung in Methanol versetzt und 15 min bei Raumtemperatur unter Argon gerührt. Die Lösung wurde mit Essigsäure angesäuert und mit einer gesättigten Natriumbicarbonat-Lösung unter Eiskühlung neutralisiert. Anschliessend wurde die Reaktionsmischung mit Dichlormethan extrahiert. Die Dichlormethan-Extrakte wurden mit Natriumsulfat getrocknet, filtriert und im Vakuum zur Trockene eingedampft. Der Rückstand wurde in 15 ml Dichlormethan gelöst, mit 27.6 g 1.5%-igem Salzsäuregas in Methanol versetzt, und im Vakuum (223 mbar) eingedampft. Der Rückstand wurde aus Aceton kristallisiert. Man erhielt 243 mg 2-Chlor-4-(2-fluor-phenyl)-8,9-dihydro-6H-imidazo-[1,2-a]thieno[3,2-f][1,4]diazepin-8-methanol-hydrochlorid vom Smp. 142-145°C (Zers., stark hygroskopisch).

### Beispiel 3

### Herstellung von 4-Phenyl-6H-imidazo[1,2-a]thieno[2,3-f][1,4]diazepin-8-methanol-dihydrochlorid

a) Zu einer Lösung von 8.82 g 5-Phenyl-2,3-dihydro-1H-thieno-[3,2-e][1,4]diazepin-2-on in 600 ml wasserfreiem Tetrahydrofuran wurden 5.46 g Kalium-tert-butylat gegeben und die Lösung 1 h bei Raumtemperatur und 1 h bei 50°C gerührt. Nach Abkühlen wurde eine Lösung von 12.72 g Dimorpholin-4-yl-phosphinchlorid in 300 ml wasserfreiem Tetrahydrofuran zugegeben und die Reaktionsmischung 20 min bei Raumtemperatur gerührt. Die Lösung wurde auf Natriumbicarbonat/ Eis/Dichlormethan gegossen, extrahiert, die Dichlormethan-Extrakte mit Natriumsulfat getrocknet, filtriert und die organische Phase im Vakuum zur Trockene eingedampft. Nach Kristallisation des Rückstandes aus Diethylether/ Dichlormethan erhielt man 2 g des Ausgangsproduktes zurück. Die Mutterlaugen wurden eingeengt und der Rückstand (Dimorpholin-4-yl-phosphinsäure-5-phenyl-3H-thieno[3,2-e][1,4]diazepin-2-yl-ester) wurde ohne weitere Reinigung als Ausgangsprodukt für die untenstehend beschriebene Stufe verwendet.
b) In eine Lösung von 14.4 g Dimorpholin-4-yl-phosphinsäure-(5-phenyl-3H-thieno[3,2-e][1,4]diazepin-2-yl)-ester wurde bei Raumtemperatur unter Rühren so lange gasförmiges Ammoniak eingeleitet, bis das Ausgangsprodukt ganz umgesetzt war. Nach Entfernen des Lösungsmittels im Vakuum wurde der Rückstand mit Dichlormethan/Wasser in Gegenwart von wenig Methanol extrahiert. Die wässerige Phase wurde mehrmals mit Dichlormethan gewaschen, die Dichlormethan-Extrakte mit Natriumsulfat getrocknet, filtriert und im Vakuum eingedampft. Der Rückstand wurde in Dichlormethan suspendiert und während 0.5 h gerührt. Man erhielt 2.9 g rohes 5-Phenyl-3H-thieno[3,2-e][1,4]diazepin-2-ylamin, welches ohne weitere Reinigung als Ausgangsprodukt für die untenstehend beschriebene Stufe verwendet wurde.
c) Eine Suspension von 2.9 g 5-Phenyl-3H-thieno[3,2-e][1,4]diazepin-2-ylamin, 11.7 g Natriumbicarbonat und 1.83 g 1,3-Dichloraceton in 80 ml Dioxan wurde während 48 h unter Argon bei Raumtemperatur gerührt. Danach wurde die Temperatur der Reaktionsmischung auf 80°C erhöht und die Suspension während weiteren 27 h unter Argon gerührt. Nach Abkühlen auf 60°C wurden 19.2 g 2,5 N Natriumhydroxid-Lösung zugegeben und die Reaktionsmischung unter Argon 20 h weitergerührt. Anschliessend wurden in zwanzigstündigen Abständen noch zweimal 19.2 g 2.5 N Natriumhydroxid-Lösung zugegeben. Die Suspension wurde mit Dichlormethan/gesättigter Natriumchlorid-Lösung in der Gegenwart von wenig Methanol extrahiert. Die Dichlormethan-Extrakte wurden mit Natriumsulfat getrocknet, filtriert und im Vakuum eingedampft. Der Rückstand wurde in Methanol zweimal hintereinander suspendiert und verrührt. Man erhielt 2.14 g rohes 4-Phenyl-6H-imidazo[1,2-a]thieno[2,3-f][1,4]-diazepin-8-ylmethanol, welches ohne weitere Reinigung als freie Base für die Herstellung des Dihydrochlorides eingesetzt wurde.
   200 mg rohes 4-Phenyl-6H-imidazo[1,2-a]thieno[2,3-f][1,4]diazepin-8-methanol wurden in heissem Methanol gelöst, heiss filtriert und mit 30 g 1.5%-iger Salzsäure in Methanol versetzt und im Vakuum (223 mbar) eingedampft. Der Rückstand wurde aus Methanol kristallisiert. Man erhielt 4-Phenyl-6H-imidazo[1,2-a]thieno[2,3-f][1,4]diazepin-8-methanol-dihydrochlorid vom Smp. >230°C.

### Beispiel 4

### Herstellung von 8-Chlor-6-(2-fluorphenyl)-4H-imidazo[1,2-a][1,4]benzodiazepin-2-methanol

a) Zu einer Lösung von 27.6 g 7-Chlor-5-(2-fluorphenyl)-3H-1,4-benzodiazepin-2(1H)-thion (J.B.Hester jr., A.D.Rudzik und B.V.Bharat, J.Med. Chem., 1971, 14, 1078-1081) in 900 ml Tetrahydrofuran wurden 800 ml wässerige Ammoniaklösung (25%) gegeben und 72 h bei Raumtemperatur intensiv gerührt. Die abgetrennte organische Phase wurde im Vakuum auf ca. 50 ml eingeengt und die dabei ausgefallenen Kristalle abfiltriert. Es wurde im Vakuum bei 50°C zunächst über Calciumchlorid, dann über Phosphorpentoxid getrocknet. Es wurden 22.8 g (87%) 7-Chlor-5-(2-fluorphenyl)-3H-1,4-benzodiazepin-2-ylamin als hellgelbes Kristallpulver vom Smp. 248-249°C (Zers.) gewonnen, welches ohne weitere Reinigung für die untenstehend beschriebenen Stufen verwendet wurde.
b) Eine Suspension von 20.0 g 7-Chlor-5-(2-fluorphenyl)-3H-1,4-benzodiazepin-2-ylamin und 9.72 g 1,3-Dichloraceton in 300 ml Dioxan wurde mit 5.84 g Natriumhydrogencarbonat versetzt und 6 h unter Rückfluss erhitzt. Dann wurden 160 ml IN wässerige Natriumhydroxid-Lösung zugetropft und eine weitere Stunde gekocht. Das Reaktionsgemisch wurde im Vakuum weitgehend eingeengt, mit Wasser versetzt und dreimal mit gleichem Volumen Dichlormethan extrahiert. Die vereinigten organischen Phasen wurden über Magnesiumsulfat getrocknet, im Vakuum eingeengt und durch 1 kg Kieselgel chromatographiert. Mit Dichlormethan, welches 5% Methanol enthielt, wurden 11.3 g des gewünschten Produktes gewonnen, welches aus Essigsäureethylester/Diisopropylether umkristallisiert wurde. So wurden 10.5 g (44%) 8-Chlor-6-(2-fluorphenyl)-4H-imidazo[1,2-a][1,4]-benzodiazepin-2-methanol vom Smp. 199-201°C gewonnen.

### Beispiel 5

### Herstellung von 8-Chlor-6-(2-fluorphenyl)-1-methyl-4H-imidazo[1,2-a]-[1,4]benzodiazepin-2-methanol

a) Eine Suspension von 120g 7-Chlor-5-(2-fluorphenyl)-3H-1,4-benzodiazepin-2(1H)-thion (J.B.Hester jr., A.D.Rudzik und B.V.Bharat, J.Med. Chem., 1971, 14, 1078-1081), 150 g Kaliumcarbonat und 300 ml Iodmethan in 2.6 1 Aceton wurde unter intensivem Rühren 3 h am Rückfluß erhitzt. Der Niederschlag wurde abfiltriert und das Filtrat im Vakuum auf etwa 1/3 des ursprünglichen Volumens eingeengt. Unter Rühren wurden 500 ml Diethylether zugefügt und der Niederschlag abfiltriert. Das Filtrat wurde bis zur Trockene eingeengt und dann mit genau soviel Aceton versetzt, daß alles in Lösung ging (ca. 300 ml). Unter intensivem Rühren wurden 1.5 1 Hexan zugetropft, vom ausgefallenen 7-Chlor-5-(2-fluorphenyl)-2-methylthio-3H-1,4-benzodiazepin abfiltriert, im Vakuum eingeengt und nach Trocknen im Vakuum bei 50°C 39.2 g isoliert. Aus der eingeengten Mutterlauge wurden nach chromatographischer Reinigung an 500 g Kieselgel mit Essigsäureethylester/Hexan 1:3 weitere 17.6 g (Gesamtausbeute 45%) des gewünschten Produktes isoliert. Dieses wurde ohne weitere Reinigung für die untenstehend beschriebene Stufe verwendet.
b) Eine Lösung von 22.0 g 7-Chlor-5-(2-fluorphenyl)-2-methylthio-3H-1,4-benzodiazepin und 20.0 g L-Threonin-methylester-hydrochlorid in 200 ml Pyridin wurde 5 h bei 75°C gerührt. Das Lösungsmittel wurde im Vakuum abgezogen und der Rückstand an 800 g Kieselgel mit Dichlormethan, welches 5% Methanol enthielt, chromatographisch gereinigt. Man erhielt 16.3 g (59%) 2-[7-Chlor-5-(2-fluorphenyl)-3H-1,4-benzodiazepin-2-ylamino]-3-hydroxy-buttersäuremethylester als Diastereomerengemisch, welches ohne weitere Reinigung als Ausgangsprodukt für die untenstehend beschriebene Stufe verwendet wurde.
c) Eine Lösung von 3.8 ml Trifluoressigsäureanhydrid in 7.3 ml Dichlormethan wurde zu einer mit Trockeneis/Aceton gekühlten Lösung von 2.7 ml Dimethylsulfoxid in 18 ml Dichlormethan so zugetropft, daß die Temperatur -55°C nicht überschritt. Nach 30 min Rühren in der Kälte wurde eine Lösung von 7.54 g 2-[7-chlor-5-(2-fluorphenyl)-3H-1,4-benzodiazepin-2-ylamino]-3-hydroxy-buttersäuremethylester in 15 ml Dichlormethan so zugetropft, daß die Temperatur - 60°C nicht überschritt. Es wurde 2 h im Trockeneisbad gerührt, dann für 5 min auf -30°C gebracht und schließlich wieder im Trockeneisbad gekühlt. Nach langsamer Zugabe von 7.5 ml Triethylamin wurde zunächst noch 30 min in der Kälte, dann 30 min bei Raumtemperatur gerührt. Zur Aufarbeitung wurde mit 30 ml Wasser extrahiert. Die organische Phase wurde über Magnesiumsulfat getrocknet, im Vakuum eingeengt und an 350 g Kieselgel chromatographisch gereinigt. Mit Dichlormethan, welches 1 % Methanol enthielt, wurden Nebenprodukte eluiert. Mit Dichlormethan, welches 3% Methanol enthielt, wurden 5.56 g (74%) 2-[7-chlor-5-(2-fluorphenyl)-3H-1,4-benzodiazepin-2-ylamino]-3-oxo-buttersäuremethylester isoliert, welches ohne weitere Reinigung für die untenstehend beschriebene Stufe verwendet wurde.
d) 1.40 g 2-[7-Chlor-5-(2-fluorphenyl)-3H-1,4-benzodiazepin-2-ylamino]-3-oxo-buttersäuremethylester wurden in 10 ml Dimethylformamid aufgenommen, 100 mg p-Toluolsulfonsäure-monohydrat zugefügt und 2 h bei 80°C gerührt. Zur Aufarbeitung wurde mit 30 ml Wasser versetzt und 5 mal mit jeweils 50 ml Diethylether extrahiert. Die vereinigten organischen Phasen wurden über Magnesiumsulfat getrocknet, im Vakuum eingeengt und an 70 g Kieselgel mit Dichlormethan, welches 2 % Methanol enthielt, chromatographisch gereinigt. Es wurden 550 mg (41%) 8-Chlor-6-(2-fluorphenyl)-1-methyl-4H-imidazo[1,2-a][1,4]benzodiazepin-2-carbonsäuremethylester vom Schmp 137-138°C erhalten, die ohne weitere Reinigung für die untenstehend beschriebene Stufe verwendet wurde.
e) 3.3 ml einer 1 M Lithiumaluminiumhydrid-Lösung in Tetrahydrofuran wurde auf -75°C gekühlt und eine Lösung von 1.26 g 8-Chlor-6-(2-fluorphenyl)-1-methyl-4H-imidazo[1,2-a][1,4]benzodiazepin-2-carbonsäure-methylester in 15 ml Tetrahydrofuran zugetropft. Nach 1 h im Trockeneis/ Aceton-Bad wurde auf 0°C gebracht und eine weitere h belassen. Zur Aufarbeitung wurde unter intensivem Rühren bei 0°C 0.1 ml Wasser zugetropft, nach 5 min 0.1 ml 15 % Natriumhydroxid-Lösung zugefügt, nach weiteren 10 min schließlich 0.3 ml Wasser zugegeben. Nach 12 h wurde abfiltriert, der Rückstand in 100 ml Methanol suspendiert, 1 h gut gerührt und abfiltriert. Die vereinigten Filtrate wurden im Vakuum eingeengt und an 80 g Kieselgel mit Dichlormethan, welches 5% Methanol enthielt, chromatographisch gereinigt. Man erhielt 234 mg (20%) 8-Chlor-6-(2-fluorphenyl)-1-methyl-4H-imidazo[1,2-a][1,4]benzodiazepin-2-methanol. Zur Herstellung des Methansulfonsäure-Salzes wurden 230 mg von diesem Produkt in 10 ml Dichlormethan gelöst und 6.0 ml einer 0.1 M Methansulfonsäure-Lösung in Diethylether langsam zugetropft. Nach Abziehen des Lösungsmittels im Vakuum wurde in 10 ml Wasser aufgenommen, filtriert und lyophilisiert. Es wurden 183. mg des hygroskopischen 8-Chlor-6-(2-fluorphenyl)-1-methyl-4H-imidazo-[1,2-a][1,4]benzodiazepin-2-methanol-methansulfonat (1:2) vom Smp. 116°C isoliert.

### Beispiel 6

### Herstellung von 8-Chlor-1-diethylaminomethyl-6-(2-fluorphenyl)-4H-imidazo[1,2-a][1,4]benzodiazepin-2-methanol-hydrochlorid (1:1)

a) Eine Suspension von 17.3 g 7-Chlor-5-(2-fluorphenyl)-3H-1,4-benzodiazepin-2-ylamin, 15.3 g Dihydroxyfumarsäure-dimethylester (E.F. Hartree, J. Am. Chem. Soc., 1953, **75**, 6244) und 45.6 g Triphenylphosphin in 350 ml Tetrahydrofuran wurde auf 0° C gekühlt und eine Lösung von 26.8 ml Azodicarbonsäure-diethylester in 50 ml Tetrahydrofuran langsam zugetropft. Es wurde noch 2 h in der Kälte, dann 10 h bei Raumtemperatur gerührt. Dann wurden bei Raumtemperatur nochmals 5.0 ml Azodicarbonsäure-diethylester, 10.0 g Triphenylphosphin und 5.0 g Dihydroxyfumarsäuredimethylester zugegeben, 2 h gerührt und schließlich 5 h unter Rückfluss erhitzt. Das Lösungsmittel wurde im Vakuum entfernt, der ölige Rückstand in 500 ml Methanol aufgenommen und nach Zugabe von 200 ml 1N Natronlauge 5 Tage gerührt. Zur Aufarbeitung wurde das Methanol bei 40°C weitgehend abgezogen und der Rückstand mit 300 ml Essigsäure-ethylester extrahiert. Die wässrige Phase wurde mit 10%-iger wässeriger Salzäure auf pH 3 gebracht und der ausgefallene Niederschlag abfiltriert, getrocknet und so 16.2 g (66%) 8-Chlor-6-(2-fluorphenyl)-4H-imidazo[1,2-a][1,4]benzodiazepin-1,2-dicarbonsäure-1-methylester isoliert. Dieses wurde ohne weitere Reinigung als Ausgangsprodukt für die untenstehend beschriebene Stufe verwendet.
b) 13.0 g 8- Chlor-6-(2-fluorphenyl)-4H-imidazo[1,2-a][1,4]-benzodiazepin-1,2-dicarbonsäure-1-methylester wurden in 100 ml Tetrahydrofuran aufgenommen, 1 ml Dimethylformamid zugegeben und bei 0°C langsam 7.0 ml Oxalylchlorid zugetropft. Nach beendeter Zugabe wurde noch 30 min bei Raumtemperatur gerührt. Dann wurde unter Kühlung eine Mischung von 25 ml Pyridin und 25 ml Methanol so zugetropft, daß die Temperatur 10°C nicht überschritt. Anschließend wurde noch 1 h bei Raumtemperatur gerührt. Zur Aufarbeitung wurde im Oelpumpen-Vakuum alles Flüchtige abgezogen, der Rückstand mit 200 ml Dichlormethan aufgenommen und mit 200 ml Wasser extrahiert. Die organische Phase wurde über Magnesiumsulfat getrocknet, im Vakuum eingeengt und durch Verreiben mit Diethylether zur Kristallisation gebracht. Nach Trocknung im Hochvakuum bei 50°C wurden so 12.1 g (90%) 8-Chlor-6-(2-fluorphenyl)-4H-imidazo[1,2-a][1,4]benzodiazepin-1,2-dicarbonsäure-dimethylester vom Smp. 206-207°C gewonnen, das so direkt ohne weitere Reinigung für die nächste Stufe eingesetzt wurde.
c) Eine Lösung von 7.29 g 8-Chlor-6-(2-fluorphenyl)-4H-imidazo-[1,2-a][1,4]benzodiazepin-1,2-dicarbonsäure-dimethylester in 100 ml Tetrahydrofuran wurde auf -78°C gekühlt und 20 ml einer IM Lithiumaluminiumhydrid-Lösung in Tetrahydrofuran so zugetropft, daß die Temperatur -70°C nicht überschritt. Nach beendeter Zugabe wurde langsam auf Raumtemperatur gebracht und 1 h gerührt. Dann wurde tropfenweise mit gesättigter Seignettesalz-Lösung versetzt und 12 h gut gerührt. Das Unlösliche wurde abfiltriert, das Filtrat eingeengt und aus Isopropanol umkristallisiert. Die eingeengte Mutterlauge wurde an 300 g Kieselgel mit Dichlormethan/ Methanol/ Eisessig 90:10:2 chromatographisch gereinigt und die das Produkt enthaltenden Fraktionen nach Abziehen des Lösungsmittels nochmals aus Isopropanol umkristallisiert. So werden insgesamt 4.1 g (65%) [8-Chlor-6-(2-fluorphenyl)-2-hydroxymethyl-4H-imidazo[1,2-a][1,4]benzodiazepin-1-yl]-methanol vom Smp. 222°C isoliert.
d) Zu einer auf 0°C gekühlten Lösung von 15.0 g 8-Chlor-6-(2-fluorphenyl)-2-hydroxymethyl-4H-imidazo[1,2-a][1,4]benzodiazepin-1-methanol und 3.29 g Imidazol in 50 ml Dimethylformamid wurde tropfenweise eine Lösung von 7.29 g tert-Butyldimethylsilylchlorid in 15 ml Dimethylformamid gegeben. Es wurde auf Raumtemperatur gebracht und 12 h bei Raumtemperatur gerührt. Zur Aufarbeitung wurde im Oelpumpenvakuum bei 40°C die Lösungsmittel entfernt und der Rückstand mit 500 ml Wasser und 500 ml Dichlormethan aufgenommen. Die wässerige Phase wurde nochmals mit gleichem Volumen Dichlormethan extrahiert und die vereinigten organischen Phasen über Magnesiumsulfat getrocknet. Nach Abziehen des Lösungsmittels im Vakuum wurde der verbleibende Rückstand an 500 g Kieselgel mit einer Dichlormethan/Methanol-Mischung 98:2 chromatographisch gereinigt. Nach Umkristallisation aus Diethylether/Hexan wurden so 14.4 g (74%) 2-(tert-Butyl-dimethyl-silanyloxymethyl)-8-chlor-6-(2-fluorphenyl)-4H-imidazo[1,2-a][1,4]benzodiazepin-1-methanol vom Smp. 172 °C gewonnen. Dieses wurde ohne weitere Reinigung als Ausgangsprodukt für die untenstehend beschriebenen Stufen verwendet.
e) Zu einer Lösung von 1.0 g 2-(tert.-Butyl-dimethyl-silanyloxymethyl)-8-chlor-6-(2-fluorphenyl)-4H-imidazo[1,2-a][1,4]benzodiazepin-1-methanol in 10 ml Tetrahydrofuran wurden nacheinander und portionsweise 820 mg Tetrabrommethan und 650 mg Triphenylphosphin gegeben. Nach 2 h Rühren bei Raumtemperatur wurden weitere 160 mg Tetrabrommethan und 130 mg Triphenylphosphin zugegeben und noch einmal 2 h gerührt. Anschließend wurde das Lösungsmittel bei 40°C im Vakuum entfernt und der Rückstand zweimal mit je 10 ml Esigsäureethylester verrührt und abfiltriert. Zu den vereinigten Filtraten wurde 2.0 ml Diethylamin gegeben und diese Reaktionsmischung 10 h bei 50°C gerührt. Sodann wurde im Oelpumpenvakuum bei 40°C alles Flüchtige abgezogen, der Rückstand mit 5 ml Tetrahydrofuran und 10 ml IN Salzsäure aufgenommen und 3 h bei Raumtemperatur gerührt. Nach Zugabe von 30 ml Essigsäureethyleester wurde die abgetrennte organische Phase nochmals mit 10 ml 1N Salzsäure extrahiert. Die vereinigten wässerigen Phasen wurden mit 10 ml 2N Natriumhydroxid-Lösung versetzt und dreimal mit je 30 ml Essigsäureethylester extrahiert. Trocknung der organischen Phase über Magnesiumsulfat und Abziehen des Lösungsmittels im Vakuum hinterließ 850 mg Rohprodukt, das an 80 g Kieselgel mit einer Mischung aus Dichlormethan/Methanol/Ammoniumhydroxid 140:10:1 chromatographisch gereinigt wurde. So wurden 740 mg (84%) 8-Chlor-1-diethylaminomethyl-6-(2-fluorphenyl)-4H-imidazo[1,2-a][1,4]benzodiazepin-2-methanol als kristalliner Schaum erhalten. Nach Umkristallisation aus Diethylether lag der Smp. bei 159°C. Zur Herstellung des Hydrochlorides wurden 280 mg dieses Produktes in 10 ml Dioxan gelöst und 6.23 ml 0.1 N Salzsäure zugetropft. Es wurde lyophilisiert, mit 10 ml Wasser aufgenommen, filtriert und nochmals lyophilisiert und so 8-Chlor-1-diethylaminomethyl-6-(2-fluorphenyl)-4H-imidazo[1,2-a][1,4]benzodiazepin-2-methanol-hydrochlorid (1:1) vom Smp. 130°C (Zers.) erhalten.

### Beispiel 7

### Herstellung von 8-Chlor-6-(2-fluorphenyl)-1-morpholin-4-ylmethyl- 4H-imidazo[1,2-a][1,4]benzodiazepin-2-methanol-hydrochlorid (1:1)

Zu einer Lösung von 1.0 g [2-(tert.-Butyl-dimethyl-silanyloxymethyl)-8-chlor-6-(2-fluorphenyl)-4H-imidazo[1,2-a][1,4]benzodiazepin-1-yl]-methanol in 10 ml Tetrahydrofuran wurden nacheinander und portionsweise 820 mg Tetrabrommethan und 650 mg Triphenylphosphin gegeben. Nach 2 h Rühren bei Raumtemperatur wurden weitere 160 mg Tetrabrommethan und 130 mg Triphenylphosphin zugegeben und noch einmal 2 h gerührt. Anschließend wurde das Lösungsmittel bei 40°C im Vakuum entfernt, der Rückstand zweimal mit je 10 ml Esigsäureethylester verrührt und abfiltriert. Zu den vereinigten Filtraten wurde 2.0 ml Morpholin gegeben und diese Reaktionsmischung 2 h bei 50°C gerührt. Sodann wurde im Oelpumpenvakuum bei 40°C alles Flüchtige abgezogen, der Rückstand mit 5 ml Tetrahydrofuran und 10 ml IN Salzsäure aufgenommen und 3 h bei Raumtemperatur gerührt. Nach Zugabe von 30 ml Essigsäureethyleester wurde die abgetrennte organische Phase nochmals mit 10 ml 1N Salzsäure extrahiert. Die vereinigten wässerigen Phasen wurden mit 10 ml 2N Natriumhydroxid-Lösung versetzt und dreimal mit je 30 ml Essigsäureethyleester extrahiert. Trocknung der organischen Phase über Magnesiumsulfat und Abziehen des Lösungsmittels im Vakuum hinterließ 800 mg Rohprodukt, das an 50 g Kieselgel mit einer Mischung aus Dichlormethan/Methanol/Ammoniumhydroxid 140:10:1 chromatographisch gereinigt wurde. So wurden 560 mg (62%) 8-Chlor-6-(2-fluorphenyl)-1-morpholin-4-ylmethyl-4H-imidazo[1,2-a][1,4]benzodiazepin-2-methanol als kristalliner Schaum isoliert. Nach Umkristallisation aus Dichlormethan/Diethylether lag der Smp. bei 216°C. Zur Herstellung des Hydrochlorides wurden 243 mg (0,551 mmol) dieses Produktes in 10 ml Dioxan gelöst und 5.22 ml 0.1 N Salzsäure zugetropft. Es wurde lyophilisiert, mit 10 ml Wasser aufgenommen, filtriert und nochmals lyophilisiert und so 8-Chlor-6-(2-fluorphenyl)-1-morpholin-4-ylmethyl-4H-imidazo[1,2-a][1,4]benzodiazepin-2-methanol-hydrochlorid (1:1) vom Smp. 155°C (Zers.) erhalten.

### Beispiel 8

### Herstellung von 8-Chlor-6-(2-fluorphenyl)-1-methylaminomethyl-4H-imidazo[1,2-a][1,4]benzodiazepin-2-methanol-hydrochlorid (1:1)

Zu einer Lösung von 1.0 g [2-(tert.-Butyl-dimethyl-silanyloxymethyl)-8-chlor-6-(2-fluorphenyl)-4H-imidazo[1,2-a][1,4]benzodiazepin-1-yl]-methanol in 10 ml Tetrahydrofuran wurden nacheinander und portionsweise 820 mg Tetrabrommethan und 650 mg Triphenylphosphin gegeben. Nach 2 h Rühren bei Raumtemperatur wurden weitere 160 mg Tetrabrommethan und 130 mg Triphenylphosphin zugegeben und noch einmal 2 h gerührt. Anschließend wurde das Lösungsmittel bei 40°C im Vakuum entfernt und der Rückstand zweimal mit je 10 ml Esigsäureethylester verrührt und abfiltriert. Die vereinigten Filtraten wurden zu 50 ml einer bei 0°C mit Methylamin gesättigten Tetrahydrofuran-Lösung gegeben und diese Reaktionsmischung 3 h bei Raumtemperatur gerührt. Sodann wurde im Oelpumpenvakuum bei 40°C alles Flüchtige abgezogen, der Rückstand mit 5 ml Tetrahydrofuran und 10 ml 1N Salzsäure aufgenommen und 3 h bei Raumtemperatur gerührt. Nach Zugabe von 30 ml Essigsäure-ethylester wurde die abgetrennte organische Phase nochmals mit 10 ml 1N Salzsäure extrahiert. Die vereinigten wässrigen Phasen wurden mit 10 ml 2N Natriumhydroxid-Lösung versetzt und dreimal mit je 30 ml Essigsäureethyleester extrahiert. Trocknung der organischen Phase über Magnesiumsulfat und Abziehen des Lösungsmittels im Vakuum hinterließ 600 mg Rohprodukt, das an 50 g Kieselgel mit einer Mischung aus Dichlormethan/ Methanol/Ammoniumhydroxid 200:10:1 chromatographisch gereinigt wurde. Es wurden 290 mg (37%) 8-Chlor-6-(2-fluorphenyl)-1-methylaminomethyl-4H-imidazo[1,2-a][1,4]-benzodiazepin-2-methanol als kristalliner Schaum isoliert. Nach Umkristallisation aus Dichlormethan/Diethylether lag der Smp. bei 172°C. Zur Herstellung des Hydrochlorides wurden 196 mg (0,509 mmol) dieses Produktes in 5 ml Dioxan gelöst und 4.85 ml 0.1 N Salzsäure zugetropft. Es wurde lyophilisiert, mit 20 ml Wasser aufgenommen, filtriert und nochmals lyophilisiert und so 8-Chlor-6-(2-fluorphenyl)-1-methylaminomethyl-4H-imidazo[1,2-a][1,4]benzodiazepin-2-methanol-hydrochlorid (1:1) vom Smp. 150°C (Zers.) isoliert.

### Beispiel 9

### Herstellung von 1-Allylaminomethyl-8-chlor-6-(2-fluorphenyl)-4H-imidazo[1,2-a][1,4]benzodiazepin-2-methanol-hydrochlorid (1:1)

Zu einer Lösung von 3.0 g [2-(tert.-Butyl-dimethyl-silanyloxymethyl)-8-chlor-6-(2-fluorphenyl)-4H-imidazo[1,2-a][1,4]benzodiazepin-1-yl]methanol in 10 ml Tetrahydrofuran wurden nacheinander und portionsweise 3.07 g Tetrabrommethan und 2.43 g (9.3 mmol) Triphenylphosphin gegeben. Nach 3 h Rühren bei Raumtemperatur wurde abfiltriert und mit wenig Tetrahydrofuran nachgewaschen. Zum Filtrat wurde 25 ml (333 mmol) Allylamin gegeben und diese Reaktionsmischung 2 h bei 50°C gerührt. Sodann wurde im Oelpumpenvakuum bei 40°C alles Flüchtige abgezogen, der Rückstand mit 30 ml Tetrahydrofuran und 30 ml 1N Salzsäure aufgenommen und 3 h bei Raumtemperatur gerührt. Nach Zugabe von 100 ml Essigsäure-ethylester wurde die abgetrennte organische Phase nochmals mit 30 ml 1N Salzsäure extrahiert. Die vereinigten wässerigen Phasen wurden mit 30 ml 2N Natriumhydroxid-Lösung versetzt und dreimal mit je 100 ml Essigsäureethylester extrahiert. Trocknung der organischen Phase über Magnesiumsulfat und Abziehen des Lösungsmittels im Vakuum hinterließ 2.6 g Rohprodukt, das an 300 g Kieselgel mit einer Mischung aus Dichlormethan/Methanol/Ammoniumhydroxid 200:10:1 chromatographisch gereinigt wurde. So wurden 1.96 g (77%) 1-Allylaminomethyl-8-chlor-6-(2-fluorphenyl)-4H-imidazo[1,2-a][1,4]benzodiazepin-2-methanol als kristalliner Schaum isoliert. Nach Umkristallisation aus Dichlormethan/ Diethylether lag der Schmelzpunkt bei 173°C. Zur Herstellung des Hydrochlorides wurden 776 mg von diesem Produkt in 30 ml Dioxan gelöst und 18.9 ml 0.1 N Salzsäure zugetropft. Es wurde lyophilisiert, mit 10 ml Wasser aufgenommen, filtriert, nochmals lyophilisiert und so 1-Allylaminomethyl-8-chlor-6-(2-fluorphenyl)-4H-imidazo[1,2-a][1,4]benzodiazepin-2-methanol-hydrochlorid (1:1) vom Smp. 140°C erhalten.

### Beispiel 10

### Herstellung von 8-Chlor-6-(2-fluorphenyl)-4H-imidazo[1,2-a][1,4]benzodiazepin-2-methanol

a) Zu einer Suspension von 32.3 g 7-Chlor-5-(2-fluorphenyl)-3H-1,4-benzodiazepin-2(1H)-thion (J.B.Hester jr., A.D.Rudzik und B.V.Bharat, J.Med. Chem., 1971, 14, 1078-1081) in 900 ml Butanol wurden 17,2 g 3,3-Diethoxy-1-hydroxy-2-propylamin (S.David und A.Veyrieres, Carbohyd.Res. 1969, 10, 35-38) zugegeben und 24 h unter Rühren am Rückfluss erhitzt. Das Lösungsmittel wurde sodann im Vakuum entfernt. Der Rückstand (55 g rotes Oel) wurde in einem 1:1-Gemisch von Essigsäure-ethylester und Hexan gelöst und durch 700 g Kieselgel chromatographiert. Mit Essigsäure-ethylester/Hexan 1:1 und 3:1 eluierte man zunächst ca 8 g eines Nebenproduktes und dann, mit Essigsäure-ethylester/Hexan 9:1, 30,8 g 7-Chlor-2-(3,3-diethoxy-1-hydroxy-2-propylamino)-5-(2-fluorphenyl)-3H-1,4-benzodiazepin, welches ohne weitere Reinigung als Ausgangsprodukt für die untenstehend beschriebene Stufe verwendet wurde.
b) Eine Lösung von 30 g 7-Chlor-2-(3,3-diethoxy-1-hydroxy-2-propylamino)-5-(2-fluorphenyl)-3H-1,4-benzodiazepin in 300 ml Eisessig wurde 5 h auf Rückflusstemperatur erhitzt. Nach Abkühlen wurde das Lösungsmittel im Vakuum entfernt, der Rückstand wurde in 300 ml Ethanol aufgenommen, die Lösung wurde mit 150 ml 2 N wässeriger Natriumhydroxyd-Lösung versetzt und 2 h bei Raumtemperatur stehen gelassen. Dann wurden ca. 200 ml Ethanol bei 30°C im Vakuum abdestilliert. Die verbleibende Lösung wurde dreimal mit Chloroform extrahiert. Die Chloroform-Extrakte wurden mit gesättigter Natriumchlorid-Lösung gewaschen, dann über Natriumsulfat getrocknet und im Vakuum zur Trockene eingedampft. Der Rückstand (20,5 g) wurde in Chloroform gelöst und durch 500 g Kieselgel chromatographiert. Mit Chloroform, welches 0,2% bis 1,6% Ethanol enthielt, wurden Nebenprodukte (total 4,1 g) eluiert. Mit Chloroform, das 2,4% bis 20% Ethanol enthielt, wurden 13,0 g des gewünschten Produktes eluiert. Nach Umkristallisation aus Essigsäureethylester/Diethylether erhielt man 11,6 g 8-Chlor-6-(2-fluorphenyl)-4H-imidazo[1,2-a][1,4]benzodiazepin-2-methanol vom Smp. 196-199,5°C. Nach nochmaliger Umkristallisation aus Essigsäure-ethylester lag der Smp. bei 199-201°C.

### Beispiel 11

### Herstellung von 8-Chlor-6-(2-chlorphenyl)-4H-imidazo[1,2-a][1,4]-benzodiazepin-2-methanol

Eine Lösung von 9,1 g 2-Amino-7-chlor-5-(2-chlorphenyl)-3H-1,4-benzodiazepin (K. Meguro, H. Tawada & Y. Kuwada, Yakugaku Zasshi, 1973, **93**, 1253-1262) und 11,4 g 1,3-Dichloraceton in 195 ml Dioxan wurde mit 24,9 g wasserfreiem Kaliumcarbonat versetzt und 22 h bei 98°C gerührt. Nach Abkühlen wurden die anorganischen Salze abfiltriert, das Filtrat mit 0.75 g p-Toluolsulfonsäure versetzt und 2,5 h bei 98°C gerührt. Zu dieser Lösung wurden 60 ml 1N wässerige Natriumhydroxyd-Lösung und 2,0 g Aktiv-Kohle zugegeben, und 2,5 h bei 80°C gerührt. Nach Abkühlen wurde das Gemisch filtriert und das Filtrat im Vakuum eingeengt. Der Rückstand wurde in 300 ml Dichlormethan aufgenommen und die Lösung mit 200 ml 6N wässeriger Salzsäure geschüttelt. Die dabei ausgefallenen Kristalle wurden abfiltriert (4,2 g), und das Filtrat noch dreimal mit je 200 ml 6N wässeriger Salzsäure extrahiert. Die vereinigten wässerigen Extrakte wurden mit wässeriger Natriumhydroxid-Lösung schwach basisch (pH 8 bis 9) gestellt, und der Niederschlag abfiltriert. Der Filterkuchen wurde zusammen mit den früher abfiltrierten Kristallen (4,2 g) in Chloroform und in 2N wässeriger Natriumhydroxyd-Lösung aufgenommen, das Gemisch wurde klar filtriert, und die wässerige Phase wird noch zweimal mit Chloroform extrahiert. Die Chloroform-Extrakte wurden über Natriumsulfat getrocknet, im Vakuum auf 100 ml eingeengt, und durch 210 g Kieselgel chromatographiert. Mit Chloroform wurde eine kleine Menge Nebenprodukt eluiert. Das gewünschte Produkt wurde mit Chloroform, welches 1 bis 4 % Ethanol enthielt, eluiert, und aus Essigsäure-ethylester kristallisiert. Man erhielt 3,8 g 8-Chlor-6-(2-chlorphenyl)-4H-imidazo[1,2-a][1,4]benzodiazepin-2-methanol vom Smp. 174-175°C, welches in das Hydrochlorid, Smp. 257-259°C überführt wurde.

### Beispiel 12

### Herstellung von 8-Chlor-6-phenyl-4H-imidazo[1,2-a][1,4]benzodiazepin-2-methanol

Eine Lösung von 10,9 g 2-Amino-7-chlor-5-phenyl-3H-1,4-benzodiazepin (K. Meguro, H. Tawada & Y. Kuwada, Yakugaku Zasshi, 1973, **93**, 1253-1262) und 5,2 g 1,3-Dichloraceton in 200 ml Dioxan wurde mit 3,13 g wasserfreiem Natriumhydrogencarbonat versetzt und 22 h bei 98°C gerührt. Nach Abkühlen wurden die anorganischen Salze abfiltriert, das Filtrat mit 0.93 g p-Toluolsulfonsäure versetzt und 1,5 h bei 98°C gerührt. Zu dieser Lösung wurden 74,5 ml 1N wässerige Natriumhydroxyd-Lösung und 2,5 g Aktiv-Kohle zugegeben, und 2 h bei 80°C gerührt. Nach Abkühlen wurde das Gemisch durch Dicalit filtriert und das Filtrat im Vakuum eingeengt. Der Rückstand wurde in Dichlormethan und Wasser aufgenommen, und die wässerige Phase wurde noch viermal mit Chloroform extrahiert. Das Chloroform wurde im Vakuum abgedampft. Der Rückstand (ca. 8 g) wurde aus Essigsäure-ethylester kristallisert. Die Kristalle (7,3 g) wurden abfiltriert, in Dichlormethan gelöst, und durch 180 g Kieselgel chromatographiert. Das gewünschte Produkt wurde mit Dichlormethan, welches 2 bis 4 % Ethanol enthielt, eluiert, und aus Essigsäure-ethylester/Ethanol 2:1 kristallisiert. Man erhielt 3,4 g 8-Chlor-6-phenyl-4H-imidazo[1,2-a][1,4]benzodiazepin-2-methanol vom Smp. 203-204°C, welches in das Hydrochlorid, Smp. 252-254°C überführt wurde.

### Beispiel 13

### Herstellung von 8-Trifluormethyl-6-phenyl-4H-imidazo[1,2-a][1,4]-benzodiazepin-2-methanol

Eine Lösung von 10,5 g 2-Amino-7-trifluormethyl-5-phenyl-3H-1,4-benzodiazepin (K. Meguro, H. Tawada & Y. Kuwada, Yakugaku Zasshi, 1973, **93,** 1253-1262) und 4,84 g 1,3-Dichloraceton in 220 ml Dioxan wurde mit 2,72 g wasserfreiem Natriumhydrogencarbonat versetzt und 16 h bei 98°C gerührt. Nach Abkühlen wurden die anorganischen Salze abfiltriert, das Filtrat mit 0.75 g p-Toluolsulfonsäure versetzt und 2 h bei 98°C gerührt. Zu dieser Lösung wurden 60 ml 1N wässerige Natriumhydroxyd-Lösung gegeben, und 2 h bei 80°C gerührt. Nach Abkühlen wurde das Gemisch im Vakuum eingeengt. Der Rückstand wurde in Chloroform und Wasser aufgenommen, und die wässerige Phase wurde noch viermal mit Chloroform extrahiert. Das Chloroform wurde im Vakuum abgedampft. Der Rückstand wurde zweimal aus Essigsäure-ethylester umkristallisert. Man erhielt 3,15 g 8-Trifluormethyl-6-phenyl-4H-imidazo[1,2-a][1,4]benzodiazepin-2-methanol vom Smp. 193-194°C, welches in das Hydrochlorid vom Smp. 252-254°C überführt wurde.

### Beispiel 14

### Herstellung von 6-(2-Fluorphenyl)-8-nitro-4H-imidazo[1,2-a][1,4]benzodiazepin-2-methanol

a) Eine Lösung von 20,0 g 5-(2-Fluorphenyl)-7-nitro-3H-1,4-benzodiazepin-2(1H)-on (G.M.Clarke, J.B.Lee, F.J.Swinbourne & B.Williamson, J.Chem.Res.Synop. **1980**, 400) in 100 ml Hexamethylphosphorsäuretriamid wurde mit 14 g Lawesson-Reagens versetzt, und das Gemisch 70 min bei 105°C gerührt. Das Reaktionsgemisch wurde in 800 ml gesättigte wässerige Natriumhydrogencarbonat-Lösung und 800 ml Wasser gegossen. Nach 15 min Rühren bei Raumtemperatur wurde der Niederschlag abfiltriert, mit Wasser gewaschen und getrocknet. Man erhielt 20 g rohes 5-(2-Fluorphenyl)-7-nitro-3H-1,4-benzodiazepin-2(1H)-thion, welches in dieser Form für die nächste Stufe verwendet wurde.
   Ein Muster reines 5-(2-Fluorphenyl)-7-nitro-3H-1,4-benzodiazepin-2(1H)-thion vom Smp. 218-220°C wurde durch Chromatographie von 2 g Rohware durch 50 g Kieselgel, Eluieren mit Dichlormethan und Umkristallisiern aus Essigsäure-ethylester gewonnen.
b) Zu einer Lösung von 20 g 5-(2-Fluorphenyl)-7-nitro-3H-1,4-benzodiazepin-2(1H)-thion in 1 l Tetrahydrofuran wurden 350 ml 25%-ige wässerige Ammoniak-Lösung zugegeben. Die Lösung wurde 20 h bei Raumtemperatur gerührt, und sodann im Vakuum auf 200 ml konzentriert. Der kristalline Niederschlag wurde abfiltriert (12,1 g) und das Filtrat auf 50 ml konzentriert. es konnten weitere 3,1 g Kristalle abfiltriert werden. Gesamtausbeute 15,2 g 2-Amino-5-(2-fluorphenyl)-7-nitro-3H-1,4-benzodiazepin vom Smp. 225-227°C.
c) Eine Lösung von 14,9 g 2-Amino-5-(2-fluorphenyl)-7-nitro-3H-1,4-benzodiazepin und 7,65 g 1,3-Dichloraceton in 300 ml Dioxan wurde mit 5,05 g wasserfreiem Natriumhydrogencarbonat versetzt und 16 h bei 98°C gerührt. Es wurden nochmals 1,53 g 1,3-Dichloraceton und 1,0 g wasserfreies Natriumhydrogencarbonat zugegeben, und weitere 4 h bei 98°C gerührt. Nach Abkühlen wurden die anorganischen Salze abfiltriert, das Filtrat mit 1,5 g p-Toluolsulfonsäure versetzt und 2 h bei 98°C gerührt. Sodann wurde das Gemisch im Vakuum eingeengt, und der Rückstand zwischen Chloroform und Wasser verteilt. Die Chloroform-Phase wurde mit Wasser gewaschen, über Natriumsulfat getrocknet und im Vakuum eingedampft. Der ölige Rückstand wurde in Dichlormethan gelöst und durch 600 g Kieselgel chromatographiert. Mit Dichlormethan/Ethanol 99:1 wurden 13 g öliges 2-Chlormethyl-6-(2-fluorphenyl)-8-nitro-4H-imidazo[1,2-a][1,4]benzodiazepin eluiert, welches für die nächste Stufe eingesetzt wurde.

Durch Umsetzen mit wasserfreier Salzsäure in Essigsäure-ethylester erhielt man das Hydrochlorid, Smp. 251-253°C.

Eine Lösung von 12 g 2-Chlormethyl-6-(2-fluorphenyl)-8-nitro-4H-imidazo[1,2-a][1,4]benzodiazepin in 100 ml Dioxan wurde mit einer Lösung von 3,5 g Natriumcarbonat in 50 ml Wasser versetzt, und 2,5 h bei 80°C gerührt. Dann wurde das Gemisch mit wässeriger 3N Salzsäure schwach sauer (pH 6 bis 7) gestellt und im Vakuum eingedampft. Der Rückstand wurde in Chloroform aufgenommen, zweimal mit Wasser gewaschen, über Natriumsulfat getrocknet und im Vakuum eingedampft. Der Rückstand wurde in Chloroform gelöst und durch 250 g Kieselgel chromatographiert. Mit Chloroform/Ethanol 98:2 wurden kleinere Verunreinigungen, mit Chloroform/ Ethanol 98:2 wurde 6-(2-Fluorphenyl)-8-nitro-4H-imidazo[1,2-a][1,4]benzodiazepin-2-methanol eluiert. Nach Umkristallisieren aus Chloroform erhielt man 5,8 g 6-(2-Fluorphenyl)-8-nitro-4H-imidazo[1,2-a][1,4]benzodiazepin-2-methanol vom Smp. 230-231°C. Dieses wurde in das Hydrochlorid vom Smp. 239-241°C überführt.

### Beispiel 15

### Herstellung von 6-(2-Chlorphenyl)-8-nitro-4H-imidazo[1,2-a][1,4]benzodiazepin-2-methanol

a) Zu einer Lösung von 14 g 5-(2-Chlorphenyl)-7-nitro-3H-1,4-benzodiazepin-2(1H)-thion (J.B. Hester, A.D. Rudzik & B.V. Kamdar, J.Med.Chem., 1971, **14**, 1078-1081) in 700 ml Tetrahydrofuran wurden zunächst 260 ml 25%-ige wässerige Ammoniak-Lösung, und dann 50 ml Methanol zugegeben. Die Lösung wurde 20 h bei Raumtemperatur gerührt, und sodann im Vakuum auf ca. 25 ml eingeengt. Der kristalline Niederschlag wurde abfiltriert und aus Dioxan umkristallisiert. Man erhielt 13,2 g 2-Amino-5-(2-chlorphenyl)-7-nitro-3H-1,4-benzodiazepin vom Smp. 222-224°C.
b) Eine Lösung von 3,14 g 2-Amino-5-(2-chlorphenyl)-7-nitro-3H-1,4-benzodiazepin und 1,39 g 1,3-Dichloraceton in 100 ml Dioxan wurde mit 0,925 g wasserfreiem Natriumhydrogencarbonat versetzt und 20 h bei 98°C gerührt. Dann wurden nochmals 0,28 g 1,3-Dichloraceton und 0,23 g wasserfreies Natriumhydrogencarbonat zugegeben, und 3 h bei 98°C gerührt. Nach Abkühlen wurden die anorganischen Salze abfiltriert, das Filtrat mit 0.23 g p-Toluolsulfonsäure versetzt und 2 h bei 98°C gerührt. Die Lösung wurde mit Aktiv-Kohle entfärbt und durch Dicalit filtriert. Das Filtrat wurde im Vakuum eingeengt, der Rückstand in Dichlormethan gelöst und durch 65 g Kieselgel chromatographiert. Mit Dichlormethan wurde eine Verunreinigung, mit Dichlormethan/Ethanol 99:1 wurden 2,7 g 2-Chlormethyl-6-(2-chlorphenyl)-8-nitro-4H-imidazo[1,2-a][1,4]benzodiazepin eluiert, welches ohne weitere Reinigung für die weitere Umsetzung verwendet wurde.

Eine Lösung von 2,7 g 2-Chlormethyl-6-(2-chlorphenyl)-8-nitro-4H-imidazo[1,2-a][1,4]benzodiazepin in 30 ml Dioxan wurde mit einer Lösung von 0,74 g Natriumhydrogencarbonat in 10 ml Wasser versetzt, und 2,5 h bei 80°C gerührt. Das Gemisch wurde filtriert und das Filtrat mit wässeriger 3N Salzsäure neutral gestellt. Das Filtrat wurde im Vakuum eingedampft und der Rückstand wurde in Chloroform und Wasser aufgenommen. Die Chloroform-Phase wurde mit Wasser gewaschen, über Natriumsulfat getrocknet, und im Vakuum eingedampft. Der Rückstand wurde in 30 ml Essigsäure-ethylester und Diethylether kristallisiert und filtriert. Die Kristalle wurden in Dichlormethan gelöst und durch 75 g Kieselgel chromatographiert. Mit Dichlormethan/Ethanol 99:1, 98:2 und 97:3 wurden verschiedene kleinere Verunreinigungen, mit Dichlormethan/Ethanol 95:5 wurde 6-(2-Chlorphenyl)-8-nitro-4H-imidazo[1,2-a][1,4]benzodiazepin-2-methanol eluiert. Nach Umkristallisieren aus Essigsäure-ethylester erhielt man 1,4 g reines 6-(2-Chlorphenyl)-8-nitro-4H-imidazo-[1,2-a][1,4]benzodiazepin-2-methanol vom Smp. 173-174°C, welches in das Methansulfonat (Smp. 211-212°C) überführt wurde.

### Beispiel 16

### Herstellung von 8-Nitro-6-phenyl-4H-imidazo[1,2-a][1,4]benzodiazepin-2-methanol

a) Zu einer Lösung von 16 g 7-Nitro-5-phenyl-3H-1,4-benzodiazepin-2(1H)-thion (J.B. Hester, A.D. Rudzik & B.V. Kamdar, J.Med.Chem., 1971, **14**, 1078-1081) in 850 ml Tetrahydrofuran wurden 300 ml 25%-ige wässerige Ammoniak-Lösung gegeben. Die Lösung wurde 20 h bei Raumtemperatur gerührt, und sodann im Vakuum auf ca. 50 ml eingeengt. Der kristalline Niederschlag wurde abfiltriert und aus Dioxan umkristallisiert. Man erhielt 8,0 g 2-Amino-7-nitro-5-phenyl-3H-1,4-benzodiazepin, welches in dieser Form für die nächste Stufe verwendet wurde.
b) Eine Lösung von 8,0 g 2-Amino-7-nitro-5-phenyl-3H-1,4-benzodiazepin und 4,34 g 1,3-Dichloraceton in 200 ml Dioxan wurde mit 2,76 g wasserfreiem Natriumhydrogencarbonat versetzt und 20 h bei 98°C gerührt. Dann wurden nochmals 0,28 g 1,3-Dichloraceton und 0,23 g wasserfreies Natriumhydrogencarbonat zugegeben, und 3 h bei 98°C gerührt. Nach Abkühlen wurden die anorganischen Salze abfiltriert, das Filtrat mit 0,65 g p-Toluolsulfonsäure versetzt und 1 h bei 98°C gerührt. Die Lösung wurde mit Aktiv-Kohle entfärbt und durch Dicalit filtriert. Das Filtrat wurde im Vakuum eingeengt, der Rückstand in Chloroform gelöst, die Chloroform-Extrakte dreimal mit Wasser gewaschen, dann über Natriumsulfat getrocknet und durch 500 g Kieselgel chromatographiert. Mit Dichlormethan wurde eine Verunreinigung, mit Dichlormethan/ Ethanol 99:1 wurden 5,1 g 2-Chlormethyl-8-nitro-6-phenyl-4H-imidazo[1,2-a][1,4]benzodiazepin eluiert, welches ohne weitere Reinigung für die weitere Umsetzung verwendet wurde.

Eine Lösung von 5,1 g 2-Chlormethyl-8-nitro-6-phenyl-4H-imidazo-[1,2-a][1,4]benzodiazepin in 70 ml Dioxan wurde mit einer Lösung von 1,54 g Natriumhydrogencarbonat in 30 ml Wasser versetzt, und 2,5 h bei 80°C gerührt. Das Gemisch wurde filtriert und das Filtrat mit wässeriger 3N Salzsäure schwach sauer (pH 6 bis 7) gestellt. Das Filtrat wurde im Vakuum eingedampft und der Rückstand wurde in Chloroform und Wasser aufgenommen. Die Chloroform-Phase wurde mit Wasser gewaschen, über Natriumsulfat getrocknet, und im Vakuum eingedampft. Der kristalline Rückstand (3,2 g) wurde in Chloroform gelöst und durch 130 g Kieselgel chromatographiert. Mit Chloroform und mit Chloroform/Ethanol 98:2 wurden verschiedene kleinere Verunreinigungen, mit Dichlormethan/ Ethanol 97:3 und 96:4 wurde 8-Nitro-6-phenyl-4H-imidazo[1,2-a][1,4]benzodiazepin-2-methanol eluiert. Nach Umkristallisieren aus Ethanol erhielt man 2,45 g reines 8-Nitro-6-phenyl-4H-imidazo[1,2-a][1,4]benzodiazepin-2-methanol vom Smp. 250-251°C, welches in das Hydrochlorid (Smp. 295-297°C) übergeführt wurde.

### Beispiel 17

### Herstellung von 6-(2-Fluorphenyl)-8-jod-4H-imidazo[1,2-a][1,4]benzodiazepin-2-methanol

a) Zu einer Lösung von 2,3 g 5-(2-Fluorphenyl)-7-jod-3H-1,4-benzodiazepin-2(1H)-thion (A. Walser, T. Flynn, C. Mason, H.Crowley, C. Maresco, B. Yaremko & M. O'Donell, J.Med.Chem., 1991, **34**, 1209) in 120 ml Tetrahydrofuran wurden 50 ml 25%-ige wässerige Ammoniak-Lösung gegeben. Nach Zugabe von 5 ml Methanol wurde die Lösung 24 h bei Raumtemperatur gerührt, und sodann im Vakuum eingedampft. Der Rückstand wurde zwischen gesättigter wässeriger Natriumhydrogencarbonat-Lösung und Chloroform verteilt. Die wässerige Phase wurde noch dreimal mit Chloroform extrahiert. Die Chloroform-Extrakte wurden über Natriumsulfat getrocknet und im Vakuum eingedampft. Der Rückstand wurde aus Essigsäure-ethylester umkristallisiert. Man erhielt 2,73 g 2-Amino-7-jod-5-(2-fluorphenyl)-3H-1,4-benzodiazepin vom Smp. 219-221°C.
b) Eine Lösung von 1,65 g 2-Amino-7-jod-5-(2-fluorphenyl)-3H-1,4-benzodiazepin und 0,67 g 1,3-Dichloraceton in 23 ml Dioxan wurde mit 0,44 g wasserfreiem Natriumhydrogencarbonat versetzt und 20 h bei 98°C gerührt. Nach Abkühlen wurden die anorganischen Salze abfiltriert, das Filtrat mit 0,35 g p-Toluolsulfonsäure versetzt und 2 h bei 98°C gerührt. Die Lösung wurde mit Aktiv-Kohle entfärbt und durch Dicalit filtriert. Das Filtrat wurde im Vakuum eingeengt, der Rückstand in Chloroform gelöst, die Chloroform-Lösung dreimal mit Wasser gewaschen, dann über Natriumsulfat getrocknet und durch 50 g Kieselgel chromatographiert. Mit Dichlormethan wurde eine Verunreinigung, mit Dichlormethan/Ethanol 99:1 wurden 1,2 g 2-Chlormethyl-6-(2-fluorphenyl)-8-jod-4H-imidazo[1,2-a][1,4]benzodiazepin eluiert, welches ohne weitere Reinigung für die weitere Umsetzung verwendet wurde.

Eine Lösung von 1,2 g 2-Chlormethyl-6-(2-fluorphenyl)-8-jod-4H-imidazo[1,2-a][1,4]benzodiazepin in 20 ml Dioxan mit einer Lösung von 0,4 g Natriumhydrogencarbonat in 10 ml Wasser versetzt, und 2 h bei 80°C gerührt. Das Gemisch wurde filtriert und das Filtrat mit wässeriger 3N Salzsäure schwach sauer (pH 6 bis 7) gestellt. Das Filtrat wurde im Vakuum eingedampft und der Rückstand wurde in Chloroform und Wasser aufgenommen. Die Chloroform-Phase wurde mit Wasser gewaschen, über Natriumsulfat getrocknet, und im Vakuum eingedampft. Der Rückstand wurde aus Ethanol umkristallisiert. Man erhielt 0,6 g amorphes 6-(2-Fluorphenyl)-8-jod-4H-imidazo[1,2-a][1,4]benzodiazepin-2-methanol, welches in das kristalline Hydrochlorid (Smp. 274-276°C) übergeführt wurde.

### Beispiel 18

### Herstellung von 8-Brom-6-(2-pyridyl)-4H-imidazo[1,2-a][1,4]benzodiazepin-2-methanol

a) 59,5 g 7-Brom-5-(2-pyridyl)-3H-1,4-benzodiazepin-2(1H)-thion (J.B. Hester, jr., A.D. Rudzik & P.F. Von Voigtlander, J.Med.Chem., 1980, **23**, 392-402) in 800 ml Tetrahydrofuran wurden zu 1,0 1 25%-iger wässeriger Ammoniak-Lösung und 300 ml Methanol gegeben. Das Gemisch wurde 16 h bei Raumtemperatur gerührt, wobei eine Lösung entstand, welche im Vakuum auf ca. 500 ml eingeengt wurde. Die Kristalle wurden abfiltriert, mit Wasser gewaschen und getrocknet. Man erhielt 52 g 2-Amino-7-brom-5-(2-pyridyl)-3H-1,4-benzodiazepin, welches ohne weitere Reinigung für die nächste Stufe verwendet wurde. Ein aus Dioxan umkristallisiertes Muster schmolz bei 213-215°C (Zers.).
b) Eine Suspension von 52 g 2-Amino-7-brom-5-(2-pyridyl)-3H-1,4-benzodiazepin in 1,5 l Tetrahydrofuran wurde nacheinander mit 58 ml N-Ethyldiisopropylamin und 44 ml Brombrenztraubensäureethylester versetzt und 20 min bei 65°C gerührt. Dann wurden nochmals 5,8 ml N-Ethyldiisopropylamin und 4,4 ml Brombrenztraubensäure-ethylester zugegeben und weitere 20 min bei 65°C gerührt. Nach Abkühlen im Eisbad wurden 0,5 l Diethylether zugegeben, und die Kristalle abfiltriert. Man erhielt 49,5 g N-Ethyldiisopropylamin-hydrobromid. Das Filtrat wurde im Vakuum eingedampft, der Rückstand in Dichlormethan aufgenommen, und zweimal mit gesättigter wässeriger Natriumhydrogencarbonat-Lösung geschüttelt. Die organische Phase wurde über Natriumsulfat getrocknet und durch 2 kg Kieselgel chromatographiert. Mit Dichlormethan/Ethanol 197:3 und 196:4 wurden zunächst kleinere Mengen von Verunreinigungen eluiert, dann wurden mit Dichlormethan/Ethanol 97:3, 95:5 und 94:6 insgesamt 58,2 g 3-[2-Amino-7-brom-5-(2-pyridyl)-3H-1,4-benzodiazepin-1-yl]brenztraubensäureethylester eluiert. Diese wurden in 1 l Essigsäure gelöst und 3,5 h bei 100°C gerührt. Die Lösung wurde dann im Vakuum eingedampft, und der Rückstand zwischen Dichlormethan und gesättigter wässeriger Natriumhydrogencarbonat-Lösung (je 1 l) verteilt. Die Dichlormethan-Phase wurde abgetrennt, über Natriumsulfat getrocknet, und durch 2 kg Kieselgel chromatographiert. Durch Eluieren mit Essigsäure-ethylester wurden 17,2 g 8-Brom-6-(2-pyridyl)-4H-imidazo[1,2-a]-[1,4]benzodiazepin-2-carbonsäure-ethylester als dunkelrote Kristalle erhalten. Diese wurden nochmals durch 2 kg Kieselgel chromatographiert. Mit Essigsäure-ethylester/Dichlormethan 1:1 und 2:1 wurden stark gefärbte Verunreinigungen eluiert. Der gewünschte 8-Brom-6-(2-pyridyl)-4H-imidazo[1,2-a][1,4]benzodiazepin-2-carbonsäureethylester wurde mit Essigsäure-ethylester eluiert. Man erhielt 11,7 g 8-Brom-6-(2-pyridyl)-4H-imidazo[1,2-a][1,4]benzodiazepin-2-carbonsäureethylester vom Smp. 227-230°C.
c) Zu einer Suspension von 2,06 g 8-Brom-6-(2-pyridyl)-4H-imidazo-[1,2-a][1,4]benzodiazepin-2-carbonsäure-ethylester in 250 ml Tetrahydrofuran wurde bei -40°C unter kräftigem Rühren 0,38 g Lithiumaluminiumhydrid portionenweise zugegeben. Es entstand dabei eine dunkelgrüne Lösung, welche 45 min bei -7 bis -10°C gerührt wurde. Dann wurde wieder auf -35°C gekühlt und tropfenweise mit 2 ml Wasser versetzt. Nach 2 h Rühren bei Raumtemperatur wurden langsam weitere 6,0 ml Wasser zugegeben. Das Reaktionsgemisch wurde durch Dicalit klar filtriert, und das Filtrat im Vakuum eingedampft. Der Rückstand wurde zwischen 100 ml Chloroform und 50 ml gesättigter wässeriger Natriumhydrogencarbonat-Lösung verteilt. Die Chloroformphase wurde abgetrennt, über Natriumsulfat getrocknet, und im Vakuum eingedampft. Der Rückstand (1,9 g) wurde in 100 ml tert-Butanol und 50 ml Pyridin gelöst, mit 1,0 g Selendioxid versetzt und 30 min bei 65°C gerührt. Das Gemisch wurde im Vakuum eingedampft, der Rückstand in Chloroform gelöst, wobei eine kleine Menge unlösliche Verunreinigung wegfiltriert wurde. Die Lösung wurde durch 250 g Aluminiumoxid (Aktivität I) chromatographiert. Mit Dichlormethan/ Etanol 98:2 wurde eine kleinere Verunreinigung eluiert, dann wurde mit Dichlormethan/Ethanol 95:5, 90:10 und 80:20 0,95 g des gewünschten 8-Brom-6-(2-pyridyl)-4H-imidazo[1,2-a][1,4]-benzodiazepin-2-methanols eluiert. Nach Umkristallisiern aus Essigsäure-ethylester erhielt man 0,80 g 8-Brom-6-(2-pyridyl)-4H-imidazo[1,2-a][1,4]benzodiazepin-2-methanol vom Smp. 176-178°C, welches in das Hydrochlorid (Smp. über 300°C) überführt wurde.

### Beispiel 19

### Herstellung von 8-Chlor-6-(2-pyridyl)-4H-imidazo[1,2-a][1,4]benzodiazepin-2-methanol

a) Eine Lösung von 18,5 g 7-Chlor-5-(2-pyridyl)-3H-1,4-benzodiazepin-2(1H)-on (N.Ch. Hindley, T.M. McClymont & G.O.Chase, F.Hoffmann-La Roche und Co., A.G., Deutsche Offenlegungsschrift 2'233'483, 1973) in 150 ml Pyridin wurde mit 14,2 g Lawesson-Reagens versetzt, und das Gemisch 2 h bei 100°C gerührt. Nach Zugabe von 1,0 g Lawesson-Reagens wurde das Gemisch weitere 0,5 h bei 100°C gerührt, und dann im Vakuum eingedampft. Der Rückstand wurde in 500 ml gesättigte wässerige Natriumhydrogencarbonat-Lösung gegossen, und der Niederschlag wurde abfiltriert (22 g). Die roten Kristalle wurden in 100 ml siedendem Methanol suspendiert. Nach dem Abkühlen wurden 100 ml Diethylether zugegeben und die Kristalle abfiltriert. Man erhielt 18,7 g 7-Chlor-5-(2-pyridyl)-3H-1,4-benzodiazepin-2(1H)-thion vom Smp. 217-218°C.
b) Zu einer Lösung von 18,5 g 7-Chlor-5-(2-pyridiyl)-3H-1,4-benzodiazepin-2(1H)-thion in 800 ml Tetrahydrofuran wurden 300 ml 25%-ige wässerige Ammoniak-Lösung und 100 ml Methanol gegeben. Das Gemisch wurde 17 h bei Raumtemperatur gerührt, wobei eine Lösung entstand, welche im Vakuum auf ca. 200 ml eingeengt wurde. Die Kristalle wurden abfiltriert, mit Wasser gewaschen und getrocknet, in 25 ml Dioxan bei 100°C gerührt, abgekühlt und abfiltriert. Man erhielt 16,3 g 2-Amino-7-chlor-5-(2-pyridyl)-3H-1,4-benzodiazepin, welches ohne weitere Reinigung für die nächste Stufe verwendet wurde. Ein aus Dioxan umkristallisiertes Muster schmolz bei 213-215°C.
c) Eine Lösung von 16,3 g 2-Amino-7-chlor-5-(2-pyridyl)-3H-1,4-benzodiazepin und 8,4 g 1,3-Dichloraceton in 600 ml Dioxan wurde mit 5,6 g wasserfreiem Natriumhydrogencarbonat versetzt und 16 h bei 100°C gerührt. Es wurden nochmals 0.84 g Dichloraceton und 0,56 g wasserfreies Natriumhydrogencarbonat zugegeben, und weitere 8 h bei 100°C gerührt. Das Gemisch wurde im Vakuum eingedampft und der Rückstand zwischen Chloroform und Wasser verteilt. Die wässerige Phase wurde zweimal mit Dichlormethan extrahiert. Die vereinigten organischen Phasen wurden im Vakuum eingedampft. Der Rückstand wurde in Dichlormethan gelöst und durch 800 g Kieselgel chromatographiert. Mit Dichlormethan/Ethanol 197:3 wurden kleinere Verunreinigungen, mit Dichlormethan/ Ethanol 196:4 wurden 10,2 g des gewünschten Produktes eluiert. Nach Umkristallisieren aus Essigsäure-ethylester erhielt man 9,0 g 2-Chlormethyl-8-chlor-6-(2-pyridyl)-4H-imidazo[1,2-a][1,4]benzodiazepin vom Smp. 161°C.
d) Eine Lösung von 2,0 g 2-Chlormethyl-8-chlor-6-(2-pyridyl)-4H-imidazo[1,2-a][1,4]benzodiazepin in 50 ml Dioxan wurde mit einer Lösung von 0,62 g Natriumhydrogencarbonat in 18 ml Wasser versetzt, und 1,5 h bei 80°C gerührt. Das Gemisch wurde im Vakuum eingedampft. Der Rückstand wurde zwischen Dichlormethan und wässeriger Natriumhydrogencarbonat-Lösung verteilt. Die wässerige Phase wurde zweimal mit Dichlormethan extrahiert. Die Dichlormethan-Extrakte wurden über Magnesiumsulfat getrocknet, und durch 100 g Kieselgel chromatographiert. 1,7 g des gewünschten Produktes wurden mit Dichlormethan/Ethanol-Gemischen, welche 8 bis 12% Ethanol enthielten, eluiert. Nach Umkristallisation aus Essigsäure-ethylester/Dieethylether erhielt man 1,65 g 8-Chlor-6-(2-pyridyl)-4H-imidazo-[1,2-a][1,4]benzodiazepin-2-methanol vom Smp. 206-207°C, welches in das Hydrochlorid (Zers. über 300°C) übergeführt wurde.

### Beispiel 20

### Herstellung von 8-Jod-6-(2-pyridyl)-4H-imidazo[1,2-a][1,4]benzodiazepin-2-methanol

a) Zu einer Suspension von 18,7 g 2-(2-Amino-5-jodbenzoyl)pyridin (R.I. Fryer, P. Zhang & R. Rios, Synth.Commun. 1993, **23**, 985-992 ) in 760 ml Benzol wurden 10,2 ml Bromacetylbromid zugegeben und das Gemisch 20 h auf Rückflusstemperatur erhitzt. Nach dem Abkühlen werden die Kristalle abfiltriert, und diese dann in einem Gemisch von 3 l gesättigter wässeriger Natriumhyrogencarbonat-Lösung und 1 l Dichlormethan 30 min gerührt. Die wässerige Phase wurde abgetrennt und nochmals mit Dichlormethan extrahiert. Die Dichlormethan-Lösungen wurden über Natriumsulfat getrocknet und eingedampft. Der dunkelbraune, kristalline Rückstand (25 g) wurde durch 400 g Kieselgel chromatographiert. Mit Toluol/ Essigsäure-ethylester 8:1 wurden 19,1 g des gewünschten Produktes eluiert, das aus Ethanol/Diethylether umkristallisiert wurde. Man erhielt 17,6 g 2-[2-(Bromacetylamino)-5-jodbenzoyl]pyridin, welches ohne weitere Reinigung für die nächste Stufe eingesetzt wurde.
b) Eine Lösung von 5,4 g Hexamethylentetramin in 400 ml wasserfreiem Ethanol wurde mit gasförmigem Ammoniak gesättigt. Die Lösung wurde auf 80°C erwärmt, und portionenweise 17,2 g 2-[2-(bromacetylamino)-5-jodbenzoyl]pyridin zugegeben. Letzteres ging langsam in Lösung; nach 30 min bei 80°C fielen Kristalle aus. Unter weiterer Einleitung von Ammoniak wurde 4 h bei Rückflusstemperatur gerührt. Nach dem Abkühlen wurden die Kristalle abfiltriert. Man erhielt 11,2 g gelbe Kristalle. Nach Eindampfen des Filtrates wurde der Rückstand in Wasser aufgenommen. Die Kristalle wurden abfiltriert (1,3 g). Beide Kristallisate wurden aus Ethanol umkristallisiert. Man erhielt 9,7 g 7-Jod-5-(2-pyridyl)-3H-1,4-benzodiazepin-2(1H)-on, Smp. 251-252°C (Zers.).
c) Eine Lösung von 3,45 g 7-Jod-5-(2-pyridyl)-3H-1,4-benzodiazepin-2(1H)-on in 80 ml Hexamethylphosphorsäuretriamid (HMPT) wurde mit 4,24 g Lawesson-Reagens versetzt, und das Gemisch 3,5 h bei 105°C gerührt. Das Reaktionsgemisch wurde in 600 ml gesättigte wässerige Natriumhydrogencarbonat-Lösung und 21 Wasser gegossen. Nach 30 min Rühren bei Raumtemperatur wurde der Niederschlag abfiltriert. Man erhielt 3,0 g rohes, gelbes 7-Jod-5-(2-pyridyl)-3H-1,4-benzodiazepin-2(1H)-thion, Smp. 242°C, welches in dieser Form für die nächste Stufe verwendet wurde.
d) Zu einer Suspension von 3,0 g 7-Jod-5-(2-pyridiyl)-3H-1,4-benzodiazepin-2(1H)-thion in 200 ml Tetrahydrofuran wurden 60 ml 25%-ige wässerige Ammoniak-Lösung und 30 ml Methanol zugegeben. Das Gemisch wurde 16 h bei Raumtemperatur gerührt, wobei eine Lösung entstand, welche im Vakuum auf ca. 50 ml eingeengt wurde. Die Kristalle wurden abfiltriert, mit Wasser gewaschen, getrocknet, in 25 ml Dioxan bei 100°C gerührt, abgekühlt und abfiltriert. Man erhielt 2,35 g 2-Amino-7-jod-5-(2-pyridyl)-3H-1,4-benzodiazepin vom Smp. 228-229°C.
e) Eine Lösung von 2,3 g 2-Amino-7-jod-5-(2-pyridyl)-3H-1,4-benzodiazepin und 0,87 g 1,3-Dichloraceton in 65 ml Dioxan wurde mit 0,6 g wasserfreiem Natriumhydrogencarbonat versetzt und 24 h bei 100°C gerührt. Es wurden nochmals 0.09 g Dichloraceton und 0,06 g wasserfreies Natriumhydrogencarbonat zugegeben, und weitere 5 h bei 100°C gerührt. Das Gemisch wurde im Vakuum eingedampft und der Rückstand zwischen Chloroform und gesättigter wässeriger Natriumhydrogencarbonat-Lösung verteilt. Die wässerige Phase wurde dreimal mit Chloroform extrahiert. Die Chloroform-Extrakte wurden über Natriumsulfat getrocknet und durch 200 g Kieselgel chromatographiert. Mit Dichlormethan/Ethanol 197:3 wurden kleinere Verunreinigungen, mit Dichlormethan/Ethanol 195:5 wurden 1,3 g des gewünschten Produktes eluiert. Nach Umkristallisieren aus Essigsäure-ethylester erhielt man 1,21 g 2-Chlormethyl-8-jod-6-(2-pyridyl)-4H-imidazo[1,2-a][1,4]benzodiazepin vom Smp. 216°C (Zers.).
f) Eine Lösung von 1,15 g 2-Chlormethyl-8-jod-6-(2-pyridyl)-4H-imidazo[1,2-a][1,4]benzodiazepin in 40 ml Dioxan wurde mit einer Lösung von 0,28 g Natriumhydrogencarbonat in 10 ml Wasser versetzt, und 1,5 h bei 80°C gerührt. Das Gemisch wurde im Vakuum eingedampft. Der Rückstand wurde zwischen Chloroform und wässeriger Natriumhydrogencarbonat-Lösung verteilt. Die wässerige Phase wurde dreimal mit Chloroform extrahiert. Die Chloroform-Extrakte wurden über Natriumsulfat getrocknet, im Vakuum auf 30 ml eingeengt und durch 50 g Kieselgel chromatographiert. 1,05 g des gewünschten Produktes wurden mit Chloroform/Ethanol-Gemischen, welche 3 bis 7% Ethanol enthielten, eluiert. Nach Umkristallisation aus Essigsäureethylester/Diethylether erhielt man 0,955 g 8-Jod-6-(2-pyridyl)-4H-imidazo[1,2-a][1,4]benzodiazepin-2-methanol vom Smp. 206-207°C, welches in das Hydrochlorid (Zers. über 300°C) übergeführt wurde.

### Beispiel 21

### Herstellung von 2-Acetoxymethyl-8-chlor-6-(2-fluorphenyl)-4H-imidazo-[1,2-a][1,4]benzodiazepin

Zu einer Lösung von 1,36 g 8-Chlor-6-(2-fluorphenyl)-4H-imidazo-[1,2-a][1,4]benzodiazepin-2-methanol in 30 ml Pyridin wurden 0,96 ml Acetanhydrid zugetropft. Das Reaktionsgemisch wurde 70 h bei Raumtemperatur gerührt und dann im Vakuum eingedampft. Der Rückstand wurde mit gesättigter wässeriger Natriumhydrogencarbonat-Lösung versetzt und diese zweimal mit Dichlormethan extrahiert. Die Dichlormethan-Extrakte wurden über Natriumsulfat getrocknet und die Lösung durch 50 g Kieselgel chromatographiert. Mit Dichlormethan/Ethanol 197:3 wurde 1,4 g Oel eluiert, das aus Diethylether/Diisopropylether kristallisiert wurde. Man erhielt 2,1 g 2-Acetoxymethyl-8-chlor-6-(2-fluorphenyl)-4H-imidazo[1,2-a][1,4]benzodiazepin vom Smp. 134-136°C, welches in das Hydrochlorid übergeführt wurde.

## Patentansprüche

1. Hydroxymethyl-imidazodiazepine und deren Ester der allgemeinen Formel 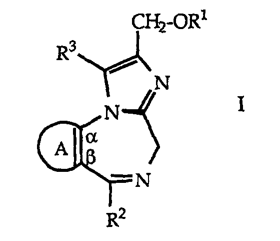 worin A zusammen mit den beiden mit α und β bezeichneten Kohlenstoffatomen einen der Reste 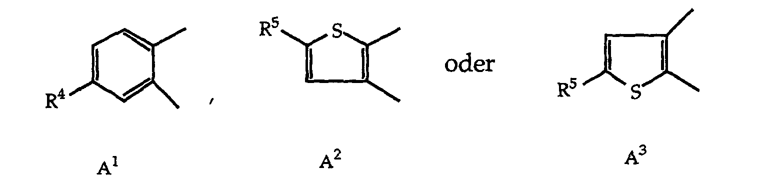
R¹ Wasserstoff oder C₁-C₇-Alkanoyl;
R² Phenyl, o-Halophenyl oder 2-Pyridyl;
R³ Wasserstoff, C₁-C₇-Alkyl, Methylaminomethyl, Allylaminomethyl oder Diethylaminomethyl:
R⁴ Halogen, CF₃ oder Nitro;
R⁵ Wasserstoff oder Halogen bedeuten, sowie Verbindung 8-Chlor-6-(2-fluorpbenyl)-1-morpholin-4ylmethyl-4H-imidazo[1,2a] [1,4]-benzodiazepin-2-methanol-hydrochlorid(1:1)
sowie pharmazeutisch annehmbare Salze von allgemeiner Formel I.

2. Verbindungen nach Anspruch 1, worin A den Rest A¹, R¹ Wasserstoff, R² o-Fluor- oder o-Chlorphenyl, R³ Wasserstoff oder Allylaminomethyl und R⁴ Chlor oder Jod bedeuten.

3. 8-Chlor-6-(2-fluorphenyl)-4H-imidazo[1,2-a]benzodiazepin-2-methanol.

4. 1-Allylaminomethyl-8-chlor-6-(2-fluorphenyl)-4H-imidazo[1,2-a]-[1,4]benzodiazepin-2-methanol.

5. 8-Chlor-6-(2-chlorphenyl)-4H-imidazo[1,2-a][1,4]benzodiazepin-2-methanol.

6. 8-Jod-6-(2-fluorphenyl)-4H-imidazo[1,2-a][1,4]benzodiazepin-2-methanol.

7. Verbindungen nach Anspruch 1, worin A den Rest A², R¹ Wasserstoff oder Carbonylmethyl, R² o-Fluorphenyl, R³ Wasserstoff und R⁵ Chlor bedeuten.

8. 2-Chlor-4-(2-fluorphenyl)-8,9-dihydro-6H-imidazo[1,2-a]thieno[3,2-f][1,4]diazepin-8-methanol.

9. Essigsäure-[2-chlor-4-(2-fluorphenyl)-8,9-dihydro-6H-imidazo[1,2-a]diazepin-8-yl]methylester.

10. Arzneimittel, insbesondere anxiolytisches und/oder antikonvulsives und/oder muskelrelaxierendes und/oder sedativ-hypnotisches Mittel, enthaltend eine Verbindung nach einem der Ansprüche 1-9 und einen therapeutisch inerten Träger.

11. Verfahren zur Herstellung von Verbindungen nach einem der Ansprüche 1-9, dadurch gekennzeichnet, dass man
a) Verbindungen der allgemeinen Formeln 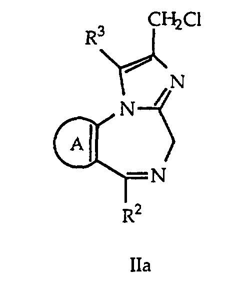 mit einem basischen Reagens zu Verbindungen der allgemeinen Formel 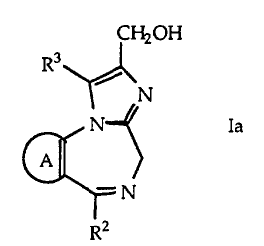 worin A, R² und R³ die obige Bedeutung haben,
umsetzt, oder
b) Verbindungen der allgemeinen Formel la oder lla zu Verbindungen der allgemeinen Formel 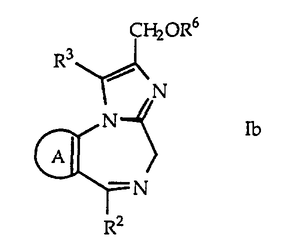 worin A, R² und R³ die obige Bedeutung haben, und R⁶ C₁-C₇-Alkanoyl bedeutet, umsetzt, oder
c) Verbindungen der allgemeinen Formel Ib zu Verbindungen der allgemeinen Formel la hydrolysiert, oder
d) Verbindungen der allgemeinen Formel 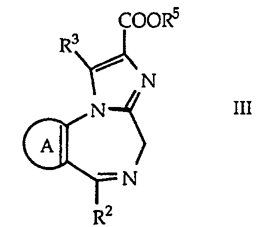 worin A, R² und R³ die obige Bedeutung haben und R⁵ C₁-C₇-Alkyl bedeutet,
zu Verbindungen der allgemeinen Formel 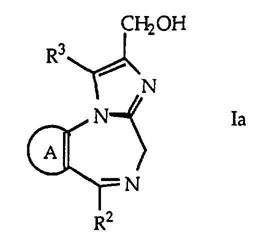 reduziert, oder
e) aus einer Verbindung der allgemeinen Formel 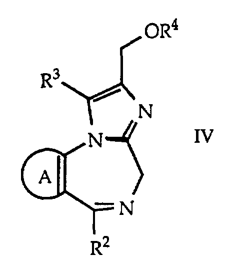 worin A, R² und R³ die obige Bedeutung haben, und R⁴ eine Schutzgruppe bedeutet, die Schutzgruppe abspaltet, oder
f) Verbindungen der allgemeinen Formel 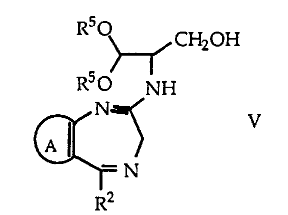 worin A und R² die obige Bedeutung haben und R⁵ C₁-C₇-Alkyl bedeutet,
im sauren Medium zu Verbindungen der allgemeinen Formel la, in welcher R³ Wasserstoff bedeutet, cyclisiert, und
g) gegebenenfalls eine Verbindung der allgemeinen Formel I in ein pharmazeutisch anwendbares Säureadditionssalz überführt.

12. Verbindungen gemäss einem der Ansprüche 1-9 zur Anwendung als therapeutische Wirkstoffe, insbesondere als anxiolytische und/oder antikonvulsive und/oder muskelrelaxierende und/oder sedativ-hypnotische Wirkstoffe.

13. Verwendung von Verbindungen nach einem der Ansprüche 1-9 für anxiolytische und/oder antikonvulsive und/oder muskelrelaxierende und/oder sedativ-hypnotische Zwecke oder zur Herstellung entsprechender Arzneimittel.

## Claims

1. Hydroxymethyl-imidazodiazepines and their esters of the general formula 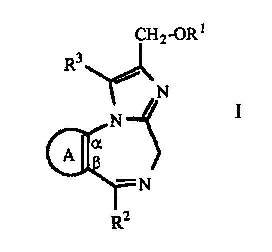 wherein A and the two carbon atoms denoted by α and β together signify one of the residues 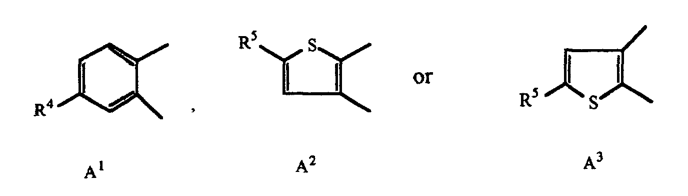
R¹ signifies hydrogen or C₁-C₇-alkanoyl;
R² signifies phenyl, o-halophenyl or 2-pyridyl;
R³ signifies hydrogen, C₁-C₇-alkyl, methylaminomethyl, allylaminomethyl or diethylaminomethyl;
R⁴ signifies halogen, CF₃ or nitro;
R⁵ signifies hydrogen or halogen, as well as the compound 8-chloro-6-(2-fluorophenyl)-1-morpholin-4ylmethyl-4H-imidazo[1,2a][1,4]benzodiazepine-2-methanol hydrochloride (1:1)
as well as pharmaceutical acceptable salts of general formula I.

2. Compound according to claim 1, wherein A signifies residue A¹, R¹ signifies hydrogen, R² signifies o-fluoro- or o-chlorophenyl, R³ signifies hydrogen or allylaminomethyl and R⁴ signifies chlorine or iodine.

3. 8-Chloro-6-(2-fluorophenyl)-4H-imidazo[1,2-a]benzodiazepine-2-methanol.

4. 1-Allylaminomethyl-8-chloro-6-(2-fluorophenyl)-4H-imidazo[1,2-a][1,4]-benzodiazepine-2-methanol.

5. 8-Chloro-6-(2-chlorophenyl)-4H-imidazo[1,2-a][1,4]benzodiazepine-2-methanol.

6. 8-Iodo-6-(2-fluorophenyl)-4H-imidazo[1,2-a][1,4]benzodiazepine-2-methanol.

7. Compounds according to claim 1, wherein A signifies residue A², R¹ signifies hydrogen or carbonylmethyl, R² signifies o-fluorophenyl, R³ signifies hydrogen and R⁵ signifies chlorine.

8. 2-Chloro-4-(2-fluorophenyl)-8,9-dihydro-6H-imidazo[1,2-a]thieno[3,2-f][1,4]diazepine-8-methanol.

9. Methyl-[2-chloro-4-(2-fluorophenyl)-8,9-dihydro-6H-imidazo[1,2-a]-diazepin-8-yl]acetate.

10. A medicament, especially an anxiolytic and/or anticonvulsant and/or muscle relaxant and/or sedative-hypnotic medicament, containing a compound according to any one of claims 1-9 and a therapeutically inert carrier.

11. A process for the manufacture of compounds according to any one of claims 1-9, characterized by
a) converting compounds of the general formulae 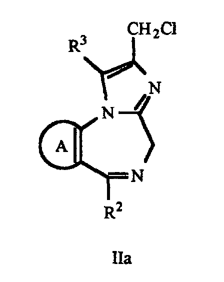 with a basic reagent into compounds of the general formula 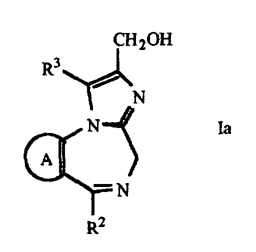 wherein A, R² and R³ have the above significance,
or
b) converting compounds of general formula Ia or IIa into compounds of the general formula 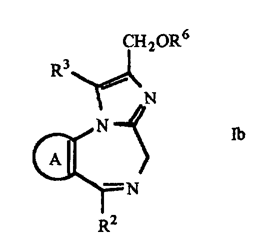 wherein A, R² and R³ have the above significance and R⁶ signifies C₁-C₇-alkanoyl,
or
c) hydrolyzing compounds of general formula Ib to compounds of general formula Ia, or
d) reducing compounds of the general formula 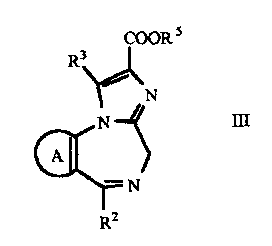 wherein A, R² and R³ have the above significance and R⁵ signifies C₁-C₇-alkyl, to compounds of the general formula 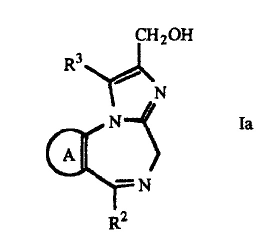 or
e) cleaving off the protecting group from a compound of the general formula 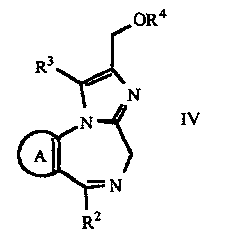 wherein A, R² and R³ have the above significance and R⁴ signifies a protecting group,
or
f) cyclizing compounds of the general formula 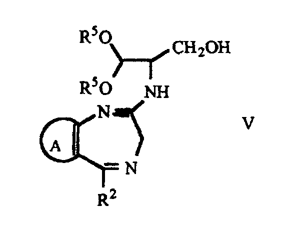 wherein A and R² have the above significance and R⁵ signifies C₁-C₇-alkyl, in an acidic medium to compounds of general formula I in which R³ signifies hydrogen, and
g) if desired, converting a compound of general formula I into a pharmaceutically usable acid addition salt.

12. Compounds in accordance with any one of claims 1-9 for use as therapeutically active substances, especially as anxiolytic and/or anticonvulsant and/or muscle relaxant and/or sedative-hypnotic active substances.

13. The use of compounds according to any one of claims 1-9 for anxiolytic and/or anticonvulsant and/or muscle relaxant and/or sedative-hypnotic purposes or for the production of corresponding medicaments.

## Revendications

1. Hydroxyméthylimidazodiazépines et leurs esters de formule générale 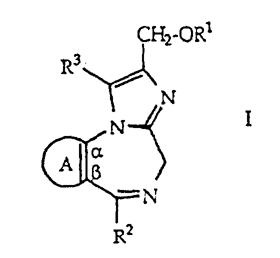 dans laquelle A avec les deux atomes de carbone désignés par α et β représentent l'un des radicaux 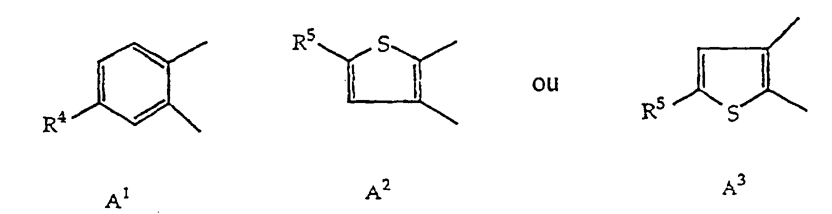
R¹ représente un hydrogène ou un alcanoyle en C₁ à C₇ ;
R² représente un phényle, un o-halophényle ou un 2-pyridyle ;
R³ représente un hydrogène, un alkyle en C₁ à C₇, un méthylaminométhyle, un allylaminométhyle ou un diméthylaminométhyle ;
R⁴ représente un halogène, CF₃ ou un nitro ;
R⁵ représente un hydrogène ou un halogène, ainsi que le composé chlorhydrate de 8-chloro-6-(2-fluorophényl)-1-morpholino-4-ylméthyl-4H-imidazo-[1,2a] [1,4]-benzodiazépine-2-méthanol (1:1)
ainsi que les sels pharmaceutiquement acceptables de formule générale (I).

2. Composés selon la revendication 1, dans lesquels A représente le radical A¹, R¹ représente un hydrogène, R² représente un o-fluoro- ou un o-chlorophényle, R³ représente un hydrogène ou un allylaminométhyle et R⁴ représente un chlore ou un iode.

3. 8-chloro-6-(2-fluorophényl)-4H-imidazo[1,2-a]benzodiazépine-2-méthanol.

4. 1-allylaminométhyl-8-chloro-6-(2-fluorophényl)-4H-imidazo[1,2-a]-[1,4]-benzodiazépine-2-méthanol.

5. 8-chloro-6-(2-chlorophényl)-4H-imidazol[1,2-a][1,4]benzodiazépine-2-méthanol.

6. 8-iodo-6-(2-fluorophényl)-4H-imidazo[1,2-a][1,4]benzodiazépine-2-méthanol.

7. Composés selon la revendication 1 dans lesquels A représente un radical A², R¹ représente un hydrogène, un carbonylméthyle, R² représente un o-fluorophényle, R³ un hydrogène et R⁵ un chlore.

8. 2-chloro-4-(2-fluorophényl)-8,9-dihydro-6H-imidazo[11,2-a]thiéno[3,2-f]-[1,4]diazépine-8-méthanol.

9. Acétate de [2-chloro-4-(2-fluorophényl)-8,9-dihydro-6H-imidazo[1,2-a]-diazépine-8-yl]méthyle.

10. Médicaments, en particulier agent à action anxiolytique et/ou anticonvulsive et/ou de relaxation musculaire et/ou sédatif hypnotique, contenant un composé selon l'une des revendications 1-9 et un support thérapeutiquement inerte.

11. Procédé de préparation de composés selon l'une des revendications 1 à 9, caractérisé en ce que
a) on fait réagir des composés de formule générale 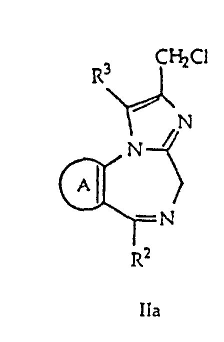 avec un réactif basique pour donner des composés de formule générale 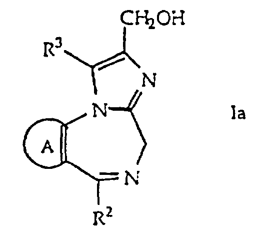 dans laquelle A, R² et R³ ont la signification indiquées ci-dessus, ou
b) on fait réagir des composés de formule générale la ou lIa pour donner des composés de formule générale 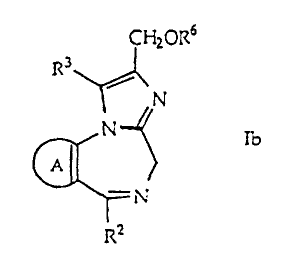 dans laquelle A, R² et R³ ont la signification donnée ci-dessus, et R⁶ représente un alcanoyl en C₁ à C₇, ou
c) on hydrolyse des composés de formule générale Ib pour donner des composés de formule générale la, ou
d) on réduit des composés de formule générale 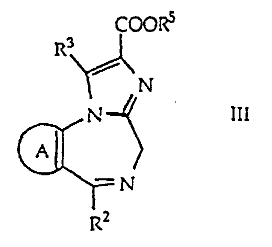 dans laquelle A, R² et R³ ont la signification donnée ci-dessus et R⁵ représente un alkyle en C¹ à C⁷ pour donner des composés de formule générale 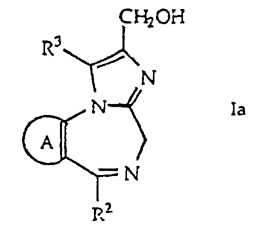 ou
e) à partir d'un composé de formule générale 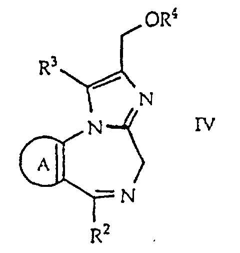 dans laquelle A, R² et R³ ont la signification donnée ci-dessus et R⁴ représente un groupe protecteur, on sépare le groupe protecteur, ou
f) on cyclise des composés de formule générale 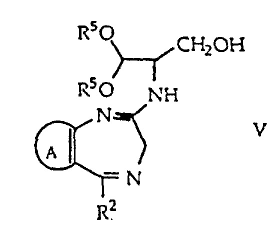 dans laquelle A et R² ont la signification donnée ci-dessus et R⁵ représente un alkyle en C₁ à C₇, en milieu acide pour donner des composés de formule générale la, dans laquelle R³ représente un hydrogène et,
g) le cas échéant on transforme un composé de formule générale I en un sel d'addition d'acide pharmaceutiquement acceptable.

12. Composés selon l'une des revendications 1 à 9 aux fins d'application comme substances actives thérapeutiques, en particulier comme substances actives anxiolytiques et/ou anticonvulsives et/ou de relaxation musculaire et/ou sédative-hypnotique.

13. Utilisation de composés selon l'une des revendications 1 à 9 comme anxiolytiques et/ou anticonvulsifs et/ou de relaxation musculaire et/ou sédatifhypnotique ou pour la fabrication de médicaments correspondants.
